(19) Europäisches Patentamt
European Patent Office
Office européen des brevets

(11) **EP 4 343 003 A1**

(12) **EUROPEAN PATENT APPLICATION**

(43) Date of publication:
**27.03.2024 Bulletin 2024/13**

(21) Application number: **22196480.2**

(22) Date of filing: **20.09.2022**

(51) International Patent Classification (IPC):
***C12Q 1/6886*** (2018.01)

(52) Cooperative Patent Classification (CPC):
**C12Q 1/6886**; C12Q 2600/118; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **HOFFMANN, Ralf Dieter
Eindhoven (NL)**

• **DHARMADHIKARI, Gitanjali
5656 AG Eindhoven (NL)**

(74) Representative: **Philips Intellectual Property & Standards
High Tech Campus 52
5656 AG Eindhoven (NL)**

Remarks:
The complete document including Reference Table(s) and the Sequence Listing(s) can be downloaded from the EPO website

(54) **PREDICTION OF AN OUTCOME OF A BLADDER CANCER SUBJECT**

(57) The invention relates to a method of predicting an outcome of a bladder cancer subject, comprising determining or receiving the result of a determination of gene expression profile comprising three or more expression levels selected from, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70 and/or PDE4D7 correlated genes selected from the group consisting of: ABCC5, CUX2, KIAA1549, PDE4D, RAP1GAP2, SLC39A11, TDRD1, and VWA2, said gene expression profiles being determined in a biological sample obtained from the subject, and determining the prediction of the outcome based on the gene expression profile.

Fig. 7

EP 4 343 003 A1

**Description**

FIELD OF THE INVENTION

The invention relates to a method of predicting an outcome of a bladder cancer subject and to a computer program product for predicting an outcome of a bladder cancer subject. Moreover, the invention relates to a diagnostic kit, to a use of the kit, to a use of the kit in a method of predicting an outcome of a bladder cancer subject, to a use of a gene expression profile of one or more immune defense response genes, one or more T-Cell receptor signalling genes and/or one or more PDE4D7-correlated genes in a method of predicting an outcome of a bladder cancer subject, and to a corresponding computer program product.

INTRODUCTION

Cancer is a class of diseases in which a group of cells displays uncontrolled growth, invasion and sometimes metastasis. These three malignant properties of cancers differentiate them from benign tumours, which are self-limited and do not invade or metastasize. Bladder cancer is a malignancy that is prevalent primarily in the developed world and is 3% of global cancer diagnoses. In the United States, bladder cancer is the 6th most prevalent cancer accounting for 4.4% of all new cancer cases [seer.cancer.gov], and the disease is four times more common in men than women. The overall prognosis of this disease is good with average 5-year survival in the US being 77%. Nevertheless, those with metastatic disease have a very low 5-year survival of 5% [Saginala et al 2020]. Depending on extent of the invasive nature of the tumour in the bladder wall, bladder cancer is classified into two principal categories: non-muscle-invasive bladder cancer (NMIBC) and muscle-invasive bladder cancer (MIBC).

NMIBC comprises nearly 75-85% of the cases and includes tumours that are confined to the mucosa and submucosa of the bladder without further muscle invasion. For determination of clinical staging, the tests involve physical examination, radiological imaging, and histological assessment of initial transurethral resection of bladder tumour (TURBT). Pathological staging is concluded after performing radical cystectomy and pelvic lymph node dissection. NMIBC comprises of 3 tumour stages as follows: Ta, papillary tumours restricted to the epithelial mucosa; Tis, carcinoma in situ (CIS), a hard to detect epithelial layer cancer that is flat or sessile; and T1, papillary tumours showing invasion of subepithelial connective tissue [Slovacek et al 2021].

The guidelines from American Urological Association (AUA), European Association of Urology (EAU), and National Institute for Health and Care Excellence (NICE) categorize NMIBC into low-risk, intermediate-risk, and high-risk groups based on the tumour grade, size, and number and recurrence rate. Prognosis is in general favourable for low as well as high grade lesions, with 10-year cancer-specific survival rates of approximately 70-85% even for high grade tumours. Recurrence occurs in 50% of patients with low-grade Ta NMIBC tumours, but only 6% of them show disease progression. In case of patients with high-grade T1 tumours, recurrence occurs in approximately 45% with the risk of cancer progression from approximately 15-40% [Slovacek et al 2021].

All NMIBC are typically removed by transurethral resection. In certain resections, a cystoscopy plus biopsy procedure may be used. The primary line of treatment for metastatic disease is platinum chemotherapy, though novel immunotherapies such as checkpoint inhibitors, are more frequently in use even as first line of treatment. In order to reduce the occurrence of relapse and progression, guidelines recommend a continued risk-stratified approach to selecting adjuvant intravesical therapy. Therapy guidelines for low-risk and intermediate-risk NMIBC recommend immediate postoperative intravesical chemotherapy and/or immunotherapy. Intravesical chemotherapy agents include mitomycin, gemcitabine, epirubicin and doxorubicin. Immunotherapy usually consists of intravesical administration of bacillus Calmette-Guerin (BCG), a live attenuated bacterium. Patients who experience recurrence after initial intravesical chemotherapy have been shown to benefit from intravesical BCG therapy, and we focus on this line of treatment to further emphasize its role.

Mycobacterium bovis Bacillus Calmette-Guérin (BCG) is used since 1970s as an immunotherapeutic treatment for bladder cancer patients to avoid recurrence and progression of the disease. The immune-mediated mechanism of action of BCG has been extensively investigated. Studies suggests that BCG binds selectively to the cell and is internalized resulting in a nonspecific immune response and subsequent tumour clearance through the activation of natural killer (NK) cells and CD8 + T cells [Rossel et al 2021, Guallar-Garrido et al 2020].

Only a single postoperative instillation is recommended for low-risk NMIBC lesions. In case of intermediate-risk NMIBC (multifocal low-grade or small-volume high-grade Ta), it is advised to opt for induction chemotherapy or immunotherapy with Bacillus Calmette Guerin (BCG), followed by maintenance therapy for 1 year in cases that are responsive to the induction. High-risk NMIBC (multifocal Ta, any T1, any CIS) are primarily treated with induction BCG, followed by maintenance therapy for up to 3 years [Slovacek et al 2021] .Owing to the recent BCG supply shortage in the USA, BCG therapy is prioritised for patients with high risk, BCG-naive NMIBC providing an induction course, followed by up to 1 year of BCG maintenance therapy at reduced dose if available, while alternative intravesical chemotherapy is listed as a primary agent for treatment of intermediate-risk disease.

Even after adequate BCG treatment, recurrence occurs in approximately 20-50% of patients. This failure of response comes from two possible scenarios as follows: (1) Prevalence of BCG intolerance in patients unable to tolerate adequate levels of BCG due to associated toxicity. (2) Presence of a subgroup of BCG-unresponsive patients. A fraction of BCG un-responsive group shows refractory disease where there is persistent high-grade NMIBC at 6 months post-treatment progression within 3 months of the first BCG treatment. In the remainder of the unresponsive group, relapse occurs after a short disease-free interval post sufficient BCG administration. [Slovacek et al 2021]. Cancers which do not respond to adequate BCG are highly unlikely to respond to additional cycles of BCG. In such patients, radical cystectomy is recommended with options of salvage intravesical therapies or enrolment in clinical trials. A cystectomy in high-grade NMIBC patients is associated with an excellent oncologic outcome, but also with high morbidity and a decreased quality of life. Furthermore, the delay in cystectomy caused by consecutive ineffective BCG re-challenges over time negatively impacts patient outcome. Unfortunately, no tools are available to predict which high-grade NMIBC patients will benefit from treatment and therefore, all patients are treated with "one-size-fits-all" BCG, leading to overtreatment and exposure to unnecessary toxicity. It would be highly beneficial to be able to stratify patients which are prognostically more suitable for BCG therapy based on additional clinical parameters of these patients like presence of biomarkers or genetic signatures of the primary tumour.

10-15% of the patients diagnosed with bladder cancer show metastasis [Rosenberg et al 2005]. More than half the patients who present with MIBC eventually die of metastatic disease. The most common metastatic sites are lymph nodes (69%), bone (47%), lung (37%), liver (26%), and peritoneum (16%) [Shinagare et al., 2011]. The more common clinical presentations of metastatic disease include occurrence of obstructive uropathy and ureteral obstruction from lymph node enlargement; lymphatic obstruction; bony involvement; lung involvement (hemoptysis, dyspnea with pleural effusion, cough), liver enzyme elevation and dysfunction; and bowel obstruction. These patients have a poor prognosis with 5-year survival rates after radical surgery are 62-68%. Neoadjuvant chemotherapy may reduce the risk of associated mortality by 33%, but accompanying toxicity also needs to be taken under consideration [Chin et al 2017]. At present, limited imaging modalities as well as poor biomarkers make it hard to have reliable prognostic predictions.

The present invention aims to overcome these issues, among other by the methods and uses as defined in the appended claims.

SUMMARY OF THE INVENTION

Non-muscle invasive bladder cancer patients have a good prognosis, but a subgroup of NMIBC patients have a high-risk of developing progression to muscle-invasive or metastatic disease. Patients with high-risk NMIBC (HR-NMIBC) are treated by transurethral resection of the bladder tumor, followed by adjuvant intravesical Bacillus- Calmette Guerin (BCG) instillations for one to three years, to decrease the probability of recurrence and progression. BCG exerts its beneficial effect by inducing a local immune response that facilitates removal of residual tumor cells. Although BCG has a high initial response rate, 17-45% of HR-NMIBC patients develop progression, leading to death in ~70% of cases within 5 years of progression diagnosis. Unfortunately, no tools are available to predict which HR-NMIBC patients will benefit from treatment and therefore, all patients are treated with BCG, leading to overtreatment and exposure to unnecessary toxicity. Similarly, treatment of patients with metastatic bladder cancer with immune checkpoint inhibitors results in a binary response with ~22% of the patients having an excellent response to the treatment, while the remaining patients have an extremely poor prognosis and appear to not respond at all to treatment. Currently non-responders cannot be predicted or identified prior to treatment, however alternative treatment strategies are ideally explored for this group. We have identified gene signatures associated with the immune system that can predict the response and survival of such patients.

In conclusion, the inventors hypothesized that there is a strong need for better prediction of the outcome of bladder cancer, and thus of a bladder cancer subject, and herein provide a method and a means of achieving an improved prediction of the outcome.

In a first aspect the invention relates to a method of predicting an outcome of a bladder cancer subject, comprising: determining or receiving the result of a determination of a gene expression profile comprising three or more gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a favourable outcome or a non-favourable outcome for the subject, optionally, providing the prediction of the outcome to a medical caregiver or the subject.

In a second aspect, the invention relates to a computer program product comprising instructions which, when the program is executed by a computer, cause the computer to carry out a method comprising: receiving data indicative of a gene expression profile comprising three or more gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from a bladder cancer subject, determining a prediction of an outcome of the subject based on the gene expression profile wherein said prediction is a favourable outcome or a non-favourable outcome.

In a third aspect the invention relates to the use of a diagnostic kit, the kit comprising: at least one polymerase chain reaction primer, and optionally at least one probes, for determining a gene expression profile, the gene expression profile comprising three or more expression levels in a biological sample obtained from a bladder cancer subject and/or in a sample, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, the use comprising predicting an outcome of a bladder cancer subject, preferably wherein the use comprises using the kit in the method as defined in the first aspect of the invention.

BRIEF DESCRIPTION OF THE DRAWINGS

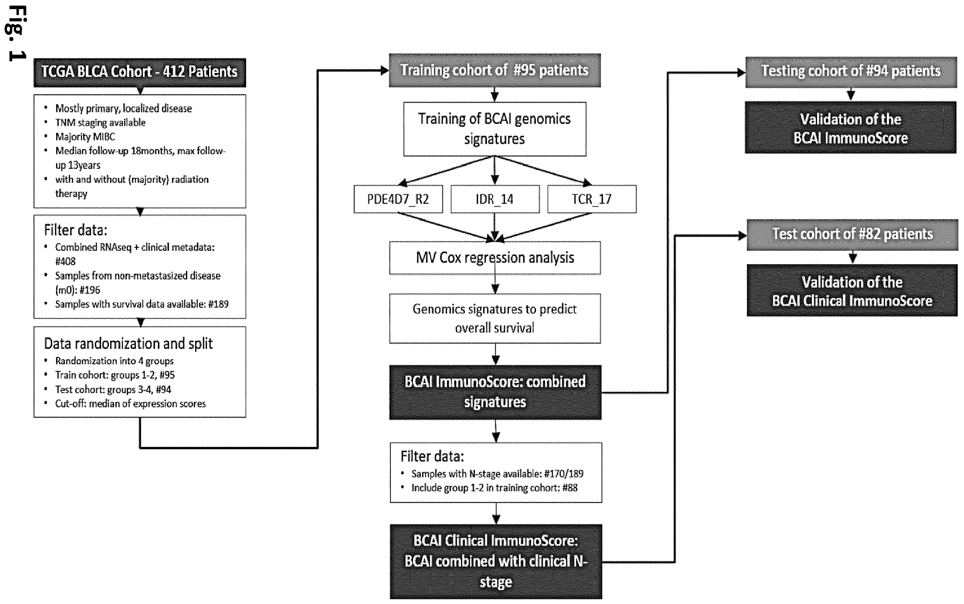

Fig 1. depicts a schematic approach to create immunoscore models for predicting an outcome for a patient with bladder cancer. Processed RNAseq data was downloaded along with clinical and demographic data from TCGA for the TCGA BLCA cohort consisting of 412 patients. The main steps in the analytics pipeline are shown in the Figure.

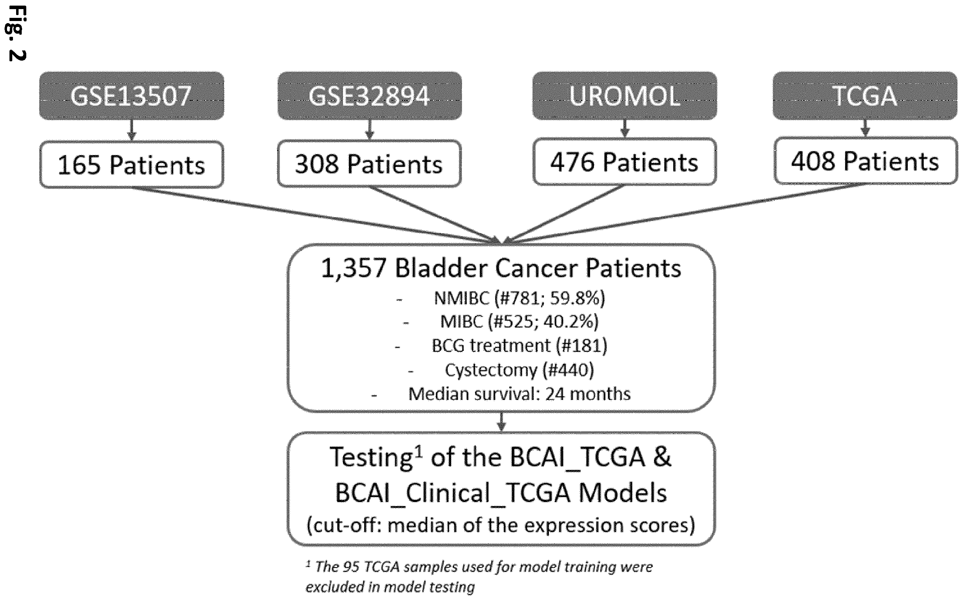

Fig. 2 depicts a schematic overview of the analytics approach to validate the immunoscore models. Creation of a super data set for bladder cancer consisting of multiple individual data sets. All BCAI relevant expression values for each data set were transformed to z scores to center the mean expression over all samples in each set to mean _expression=0 and SD_expression=1; after that normalization step the expression data for the BCAI relevant genes were combined to create the super data set comprising > 1,300 patients. Note that not for all patients all demographic or survival data was available. Consequently, the number of patients in the downstream data analysis can be less than the total number of patients in the super data set. The number of patients in each analysis are indicated for each analysis performed.

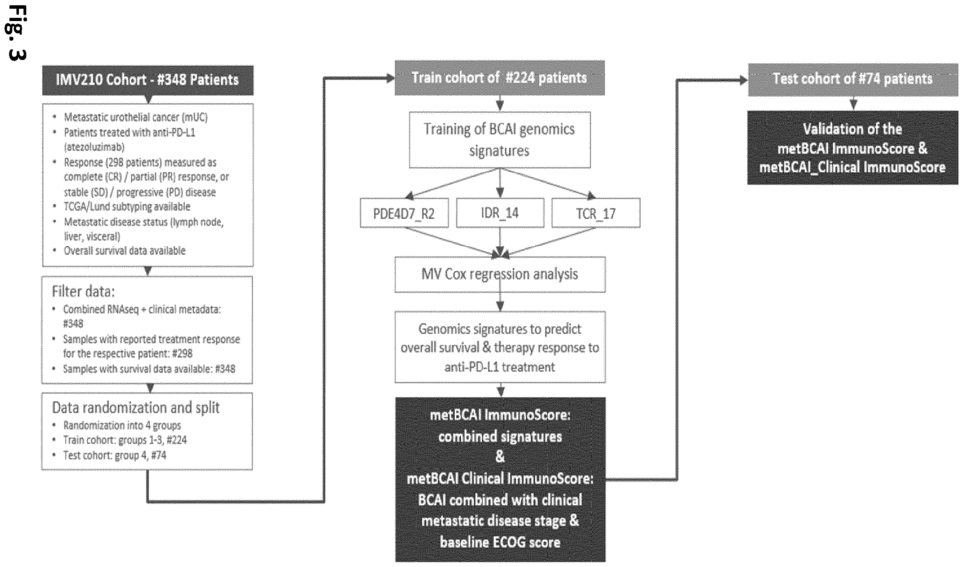

Fig. 3 depicts a schematic representation of the analytics approach to create and test the metastatic immunoscore models (mBCAI and mBCAI clinical). To test the power of the ImmunoScore in the setting of immunotherapy (anti-PD-L1; atezolizumab) treatment of metastatic bladder cancer patients (IMVigor210 trial; see reference below). To accommodate for the metastatic setting a metastatic model was re-trained on parts of the data (training data) while the models (mBCAI and mBCAI_Clinical) were tested on a validation set that was not used for training of the models.

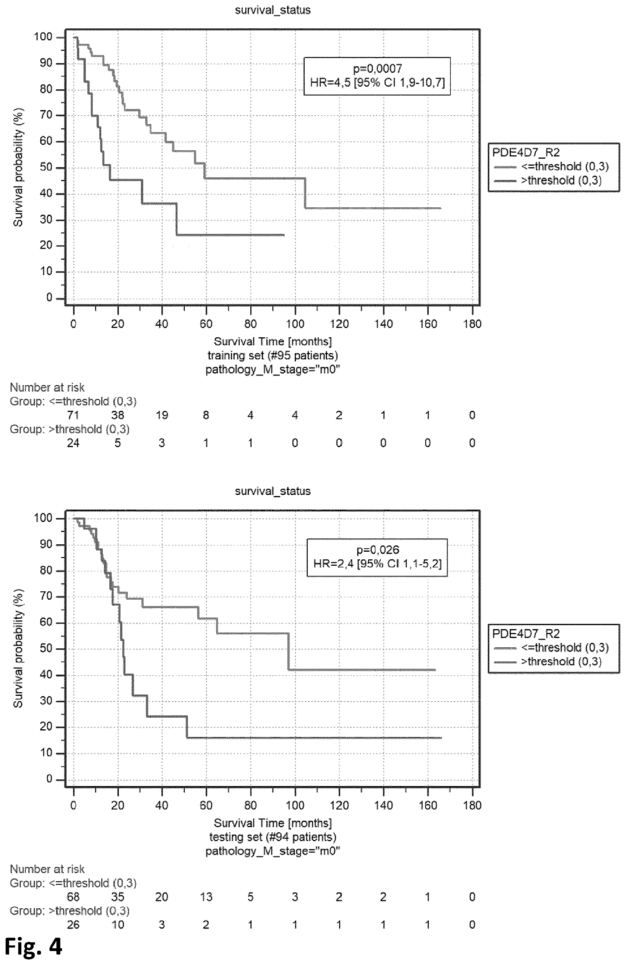

Fig. 4 depicts Kaplan-Meier survival curve analysis of the PDE4D7_R2 model. The clinical endpoint tested was time to all-cause-death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort, top graph) and were fixed for validation in the test cohort (groups 3-4 of the total cohort - bottom graph). In the validation (test) cohort, the PDE4D7_R2 Score predicts overall survival; patients classified into the high-risk group (>threshold 0.3) had a 2.4-fold increased hazard risk to die.

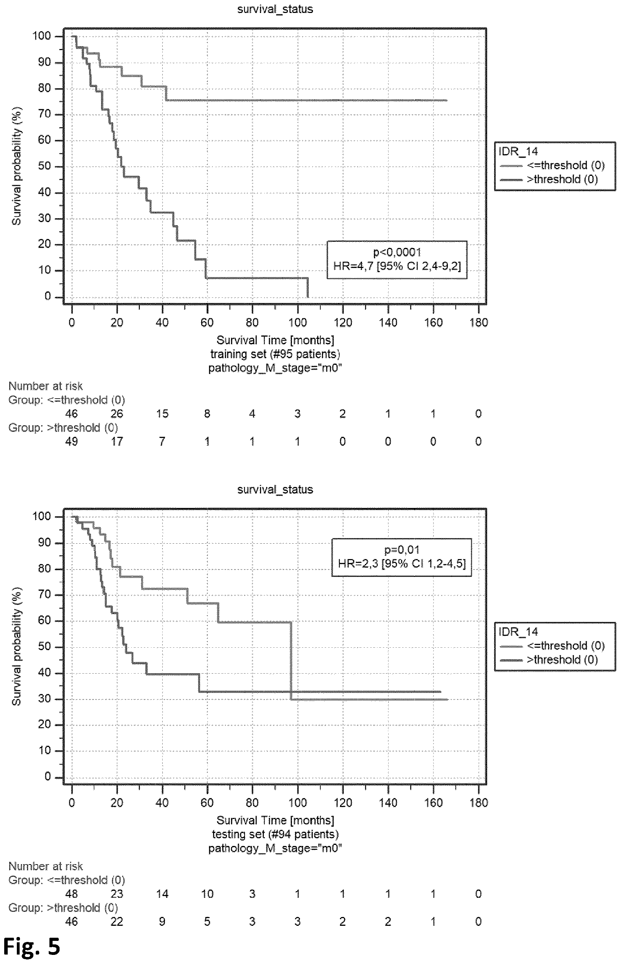

Fig. 5 Kaplan-Meier survival curve analysis of the IDR_14 model. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort - top graph) and were fixed for validation in the test cohort (groups 3-4 of the total cohort - bottom graph). In the validation (test) cohort, the IDR_14 Score predicts overall survival; patients classified into the high-risk group (>threshold 0) had a 2.3-fold increased hazard risk to die.

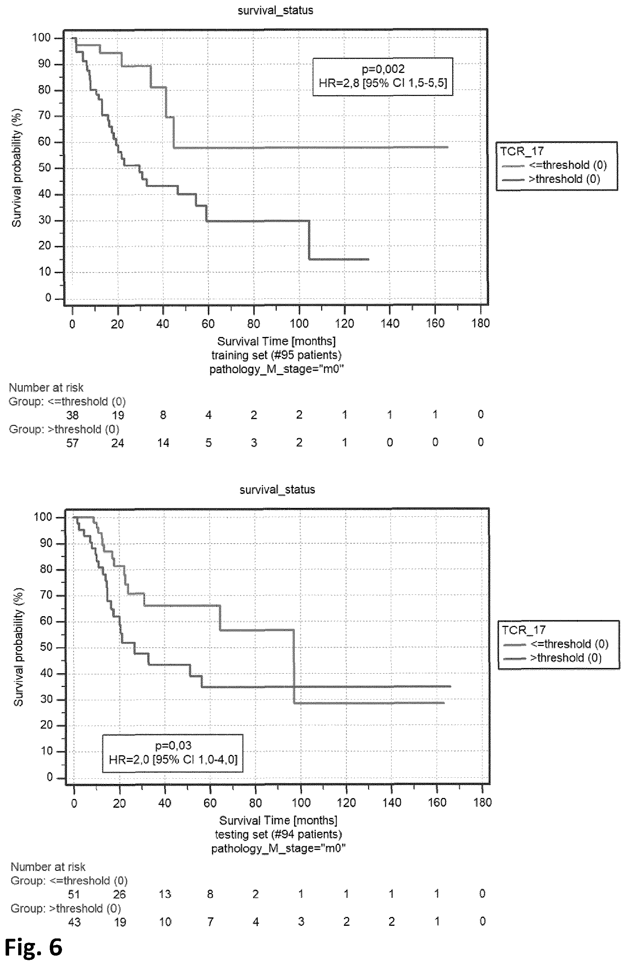

Fig. 6 Kaplan-Meier survival curve analysis of the TCR_17 model. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort, top graph) and were fixed for validation in the test cohort (groups 3-4 of the total cohort, bottom graph). In the validation (test) cohort, the TCR_14 Score predicts overall survival; patients classified into the high-risk group (>threshold 0) had a 2-fold increased hazard risk to die.

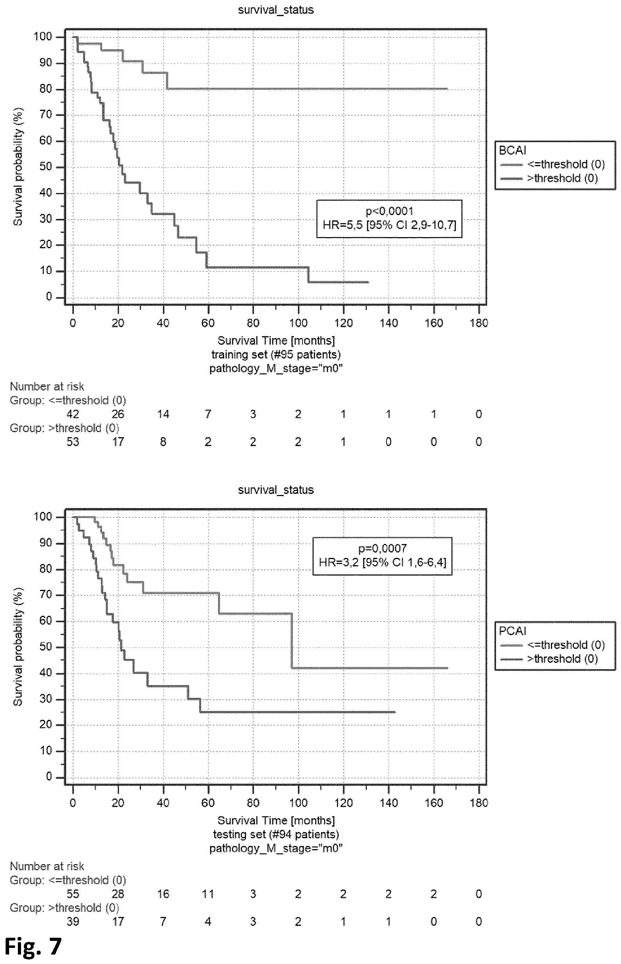

Fig. 7 Kaplan-Meier survival curve analysis of the BCAI score. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort, top graph) and were fixed for validation in the test cohort (groups 3-4 of the total cohort, bottom graph). In the validation (test) cohort, the BCAI Score predicts overall survival; patients classified into the high-risk group (>threshold 0) had a 3.2-fold increased hazard risk to die.

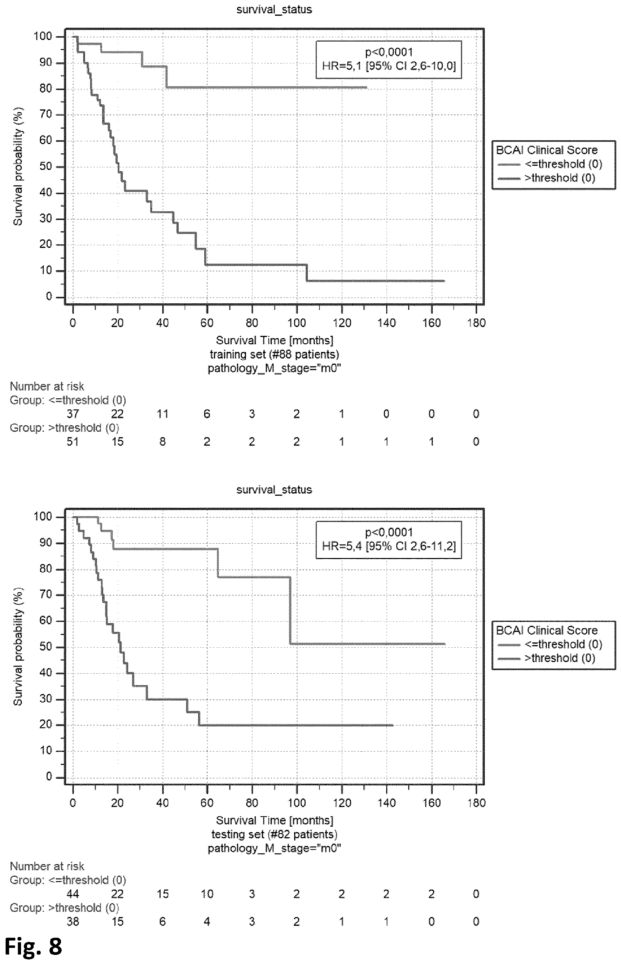

Fig 8 Kaplan-Meier survival curve analysis of the BCAI Clinical score. The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort, top graph) and were fixed for validation in the test cohort (groups 3-4 of the total cohort, bottom graph). In the validation (test) cohort, the BCAI Clinical Score predicts overall survival; patients classified into the high-risk group (>threshold 0) had a 5.4-fold increased hazard risk to die.

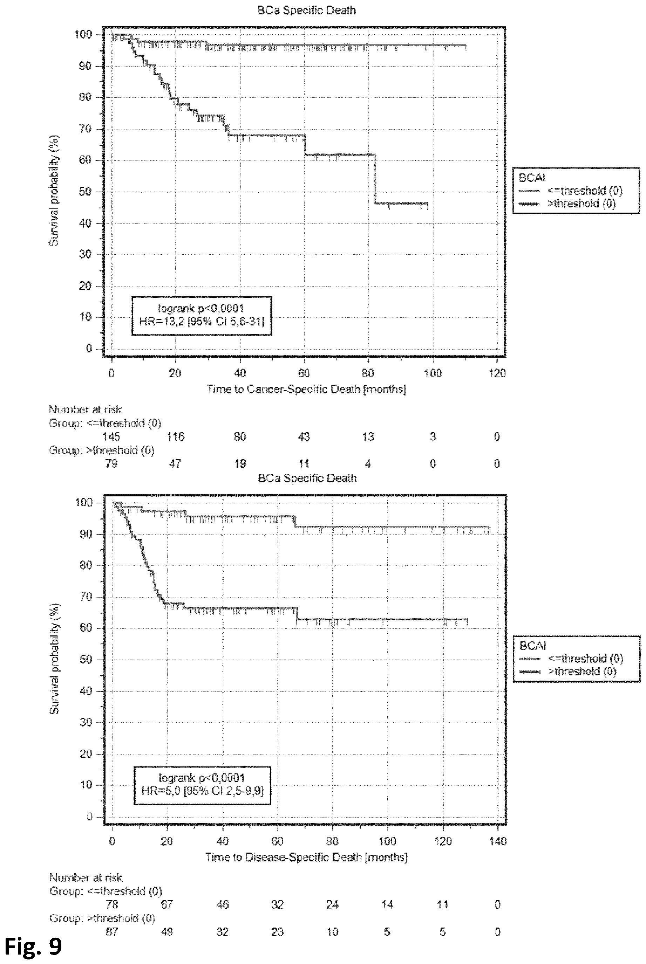

Fig. 9 Kaplan-Meier survival curve analysis of the BCAI score. The clinical endpoint tested was bladder cancer specific death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-2 of the total cohort, top graph) and were fixed for validation in the test cohort (groups 3-4 of the total cohort, bottom graph). In the validation (test) cohort, the BCAI Score predicts overall survival; patients classified into the high-risk group (>threshold 0) had a 5.0-fold increased hazard risk to die.

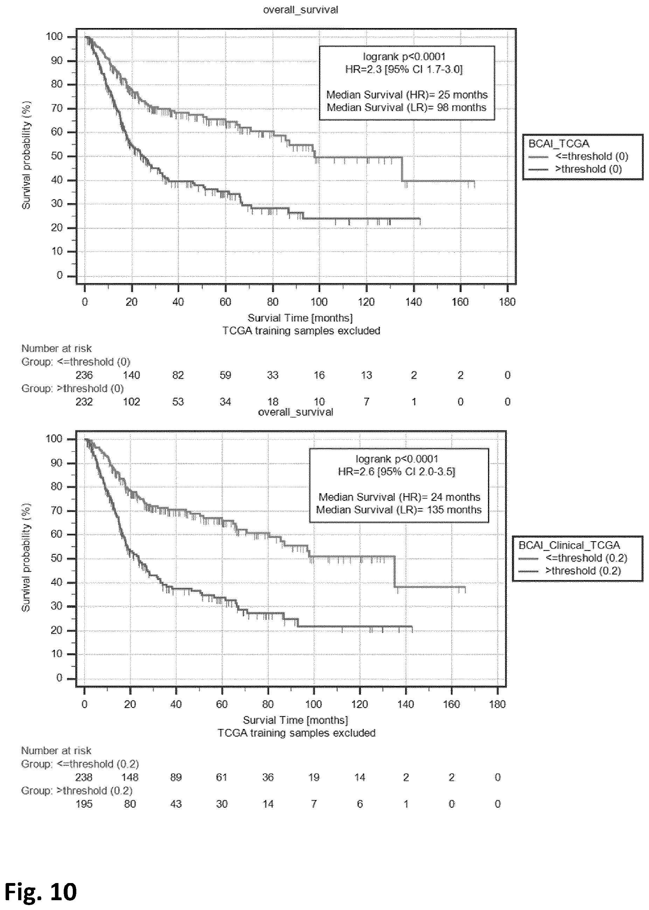

Fig. 10 The BCAI (top graph) and BCAI_Clinical (bottom graph) Cox regression models as trained on 95 TCGA data (see previous figures) were used for testing of survival endpoints (overall survival) in the entire bladder cancer cohort. The same endpoints were tested the context of different treatments for NMIBC (BCG) and MIBC (cystectomy). The respective TCGA samples which were used for model training were excluded from the testing analysis. In the validation (test) cohort, the BCAI and BCAI clinical Score predicts overall survival; patients classified into the high-risk group (>threshold 0 and 0.2 respectively) had a 2.3-fold or 2.6 fold increased hazard risk to die respectively.

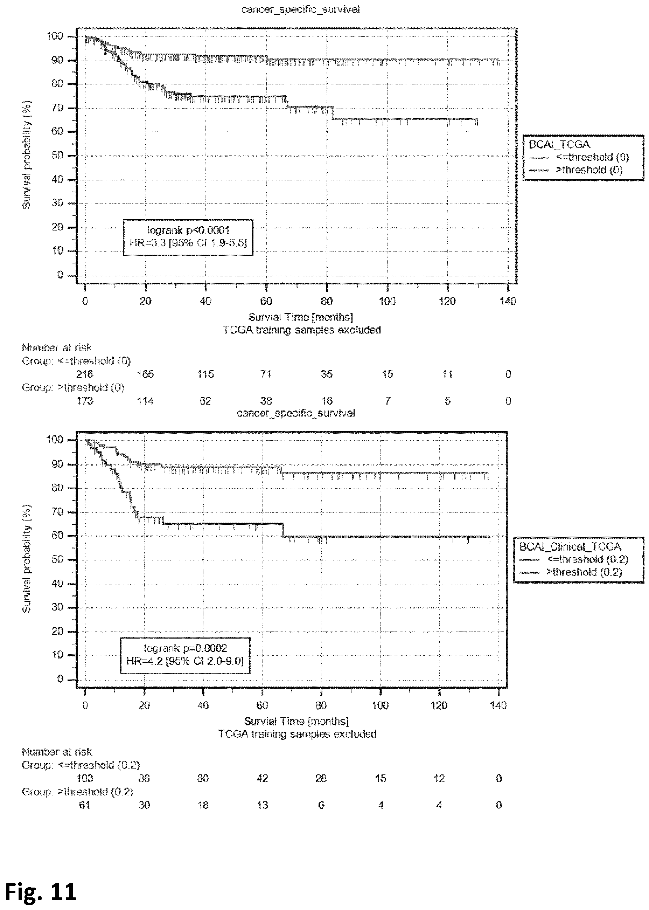

Fig. 11 The BCAI (top graph) and BCAI_Clinical (bottom graph) Cox regression models as trained on 95 TCGA data (see previous figures) were used for testing of survival endpoints (cancer specific survival) in the entire bladder cancer cohort. The respective TCGA samples which were used for model training were excluded from the testing analysis. In the validation (test) cohort, the BCAI and BCAI clinical Score predicts cancer specific survival; patients classified into the high-risk group (>threshold 0 and 0.2 respectively) had a 3.3-fold or 4.2 fold increased hazard risk to die respectively.

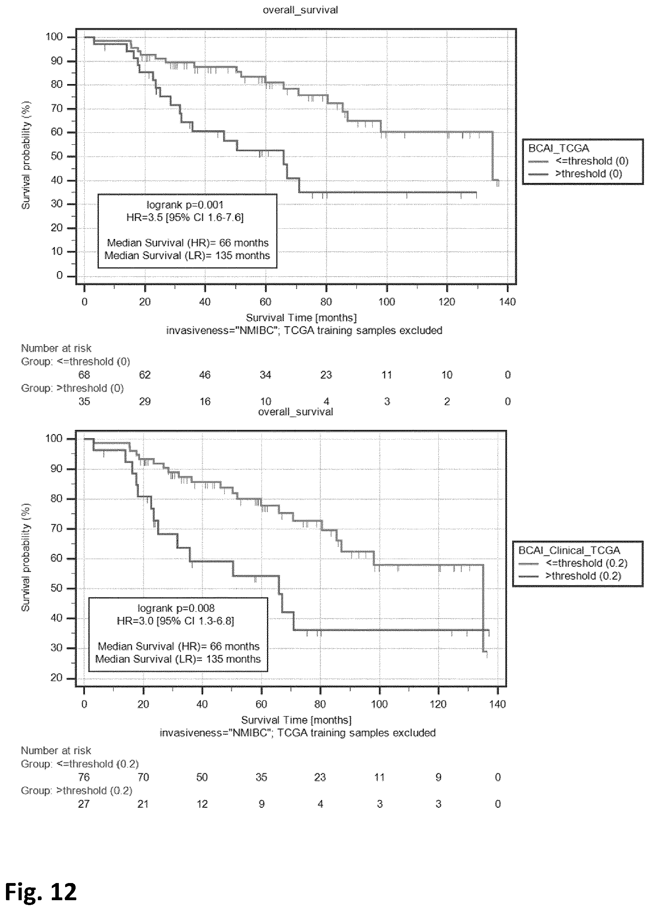

Fig. 12 The BCAI (top graph) and BCAI_Clinical (bottom graph) Cox regression models as trained on 95 TCGA data (see previous figures) were used for testing of survival endpoints (overall survival) in the NMIBC subtypes. The respective TCGA samples which were used for model training were excluded from the testing analysis. In the validation (test) cohort, the BCAI and BCAI clinical Score predicts cancer specific survival; patients classified into the high-risk group (>threshold 0 and 0.2 respectively) had a 3.5-fold or 3.0 fold increased hazard risk to die respectively.

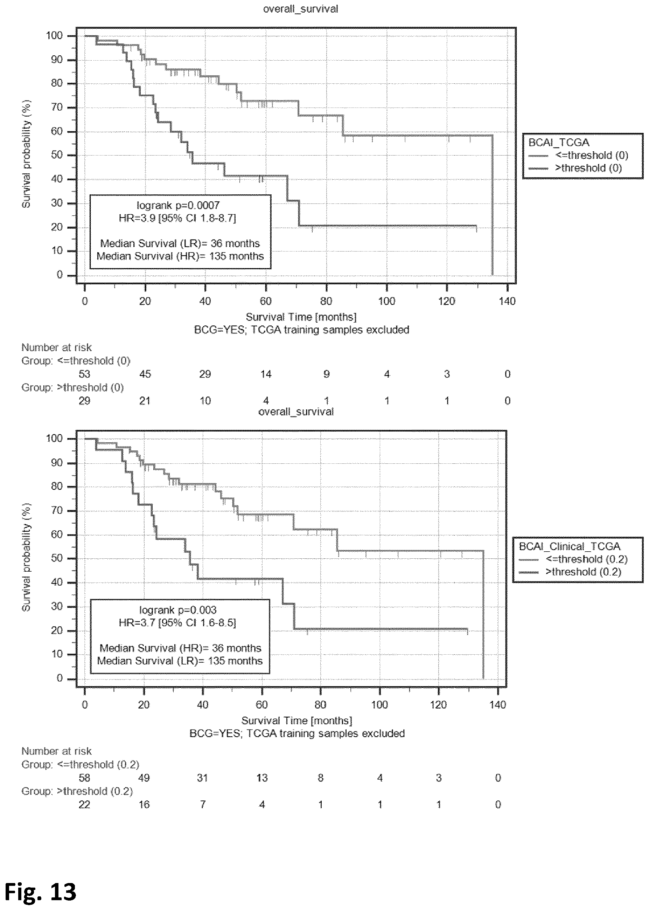

Fig. 13 The BCAI (top graph) and BCAI_Clinical (bottom graph) Cox regression models as trained on 95 TCGA data (see previous figures) were used for testing of survival endpoints (overall survival) in the NMIBC after BCG treatment subtypes. The respective TCGA samples which were used for model training were excluded from the testing analysis. In the validation (test) cohort, the BCAI and BCAI clinical Score predicts cancer specific survival; patients classified into the high-risk group (>threshold 0 and 0.2 respectively) had a 3.9-fold or 3.7 fold increased hazard risk to die respectively.

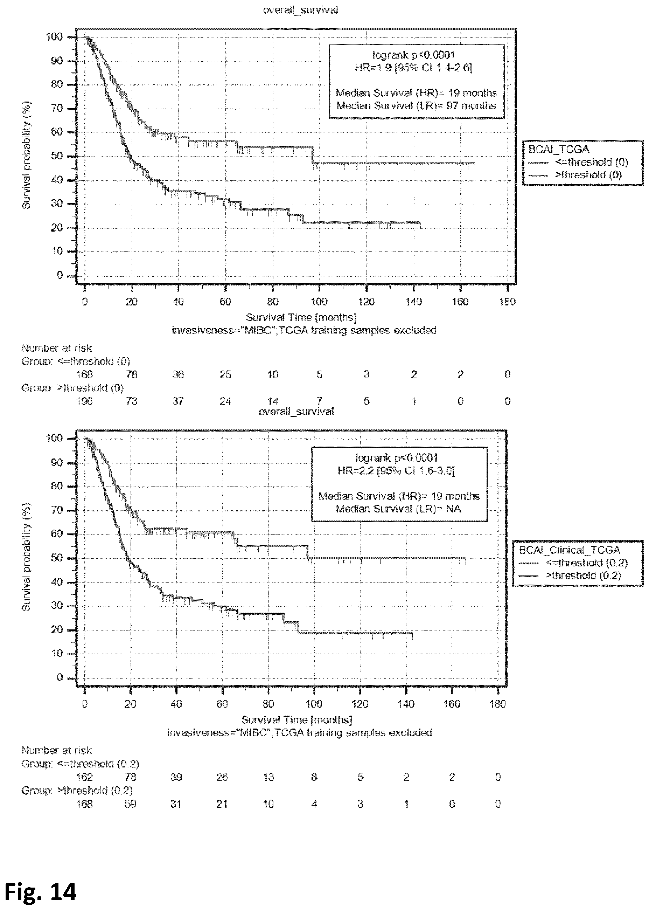

Fig. 14 The BCAI (top graph) and BCAI_Clinical (bottom graph) Cox regression models as trained on 95 TCGA data (see previous figures) were used for testing of survival endpoints (overall survival) in the MIBC subtypes. The respective TCGA samples which were used for model training were excluded from the testing analysis. In the validation (test) cohort, the BCAI and BCAI clinical Score predicts cancer specific survival; patients classified into the high-risk group (>threshold 0 and 0.2 respectively) had a 1.9-fold or 2.2 fold increased hazard risk to die respectively.

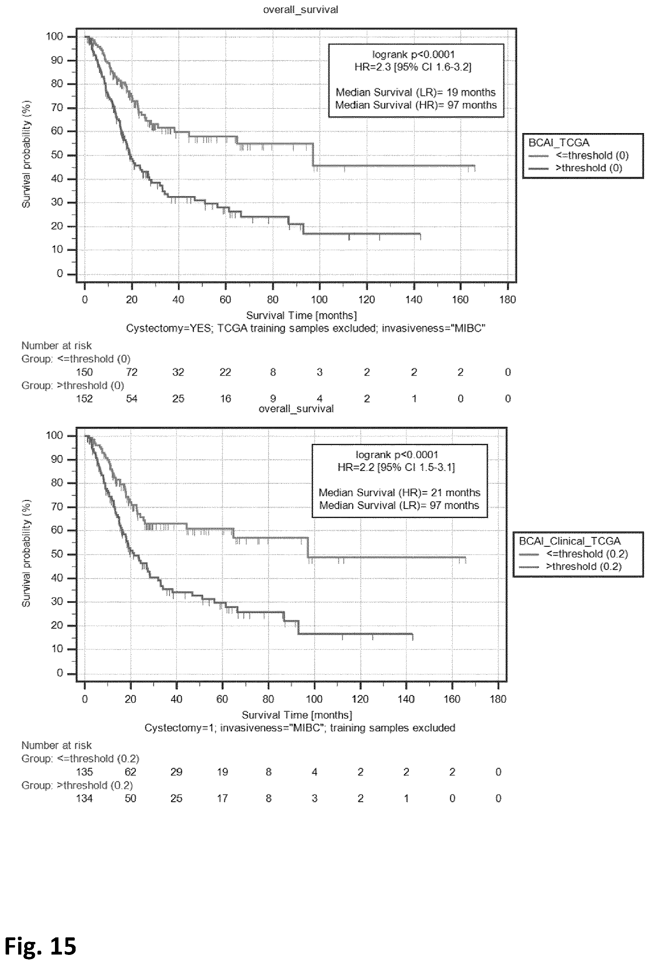

Fig. 15 The BCAI (top graph) and BCAI_Clinical (bottom graph) Cox regression models as trained on 95 TCGA data (see previous figures) were used for testing of survival endpoints (overall survival) in the MIBC after surgery (cystectomy) subtypes. The same endpoints were tested the context of different treatments for NMIBC (BCG) and MIBC (cystectomy). The respective TCGA samples which were used for model training were excluded from the testing analysis. In the validation (test) cohort, the BCAI and BCAI clinical Score predicts cancer specific survival; patients classified into the high-risk group (>threshold 0 and 0.2 respectively) had a 2.3-fold or 2.2 fold increased hazard risk to die respectively.

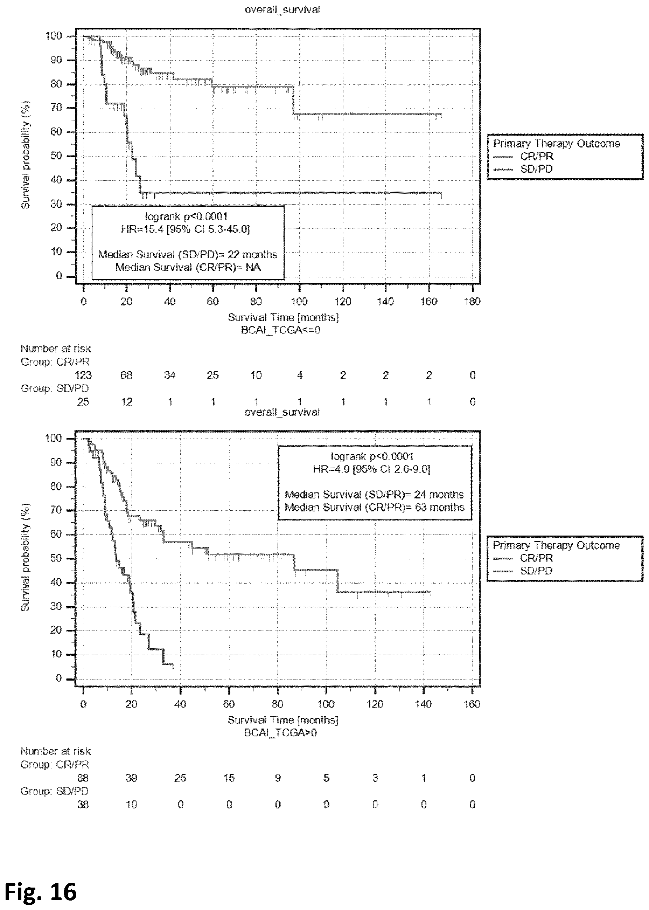

Fig. 16 The TCGA samples were divided in high and low risk based on the BCAI Immunoscore model. The high and low risk groups were then plotted in Kaplan-Meier plots (top low risk and bottom high risk groups based on BCAI stratification) based on primary therapy outcome, either Complete Response (CR) / Partial Response (PR) or Progressive Disease (PD) / Stable Disease (SD). An increased median survival can be observed in the SD/PR group for low risk (24 months vs 22 months). The median survival for CR/PR could not be calculated for the low risk group, but visually it appears that median survival is strongly increased in the CR/PR group for the low risk group.

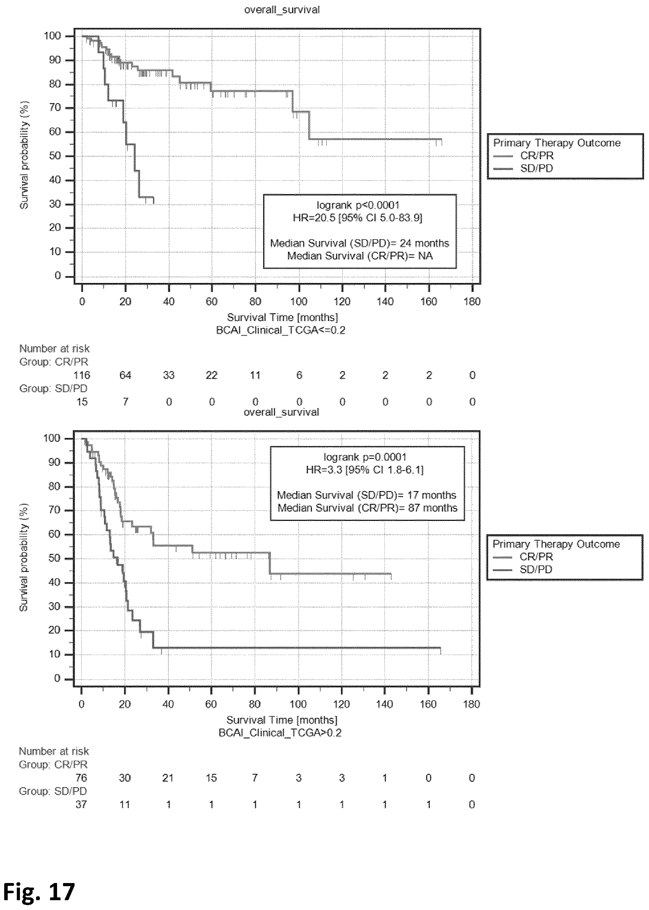

Fig. 17 The TCGA samples were divided in high and low risk based on the BCAI Clinical Immunoscore model. The high and low risk groups were then plotted in Kaplan-Meier plots (top low risk and bottom high risk groups based on BCAI Clinical stratification) based on primary therapy outcome, either Complete Response (CR) / Partial Response (PR) or Progressive Disease (PD) / Stable Disease (SD). An increased median survival can be observed in the SD/PR group for low risk (17 months vs 24 months). The median survival for CR/PR could not be calculated for the low risk group, but visually it appears that median survival is strongly increased in the CR/PR group for the low risk group.

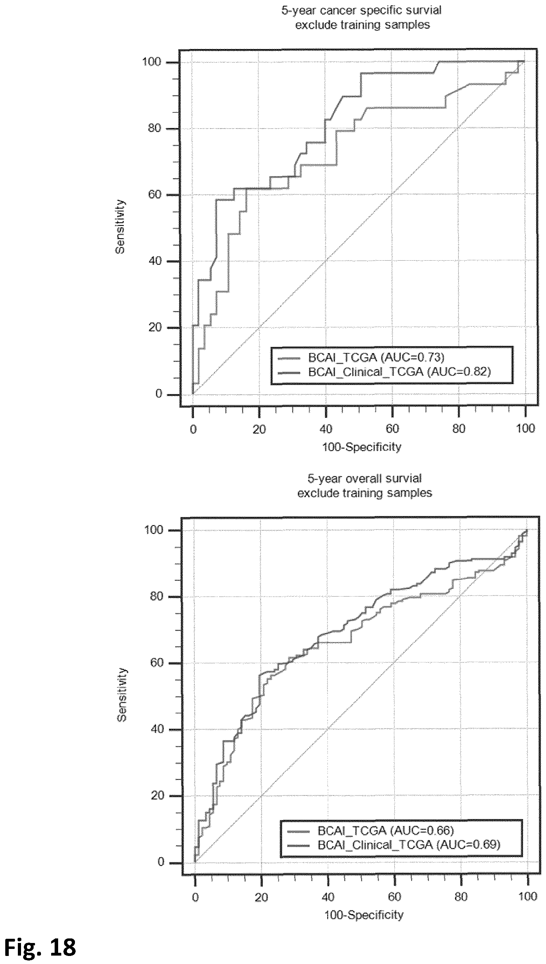

Fig. 18 depicts ROC curves for 5-year cancer specific survival (upper graph) and 5-year overall survival (lower graph). Plotted are both BCAI and BCAI clinical models as trained on 95 TCGA data. Area under curve (AUC) is indicated for each plot in the inset.

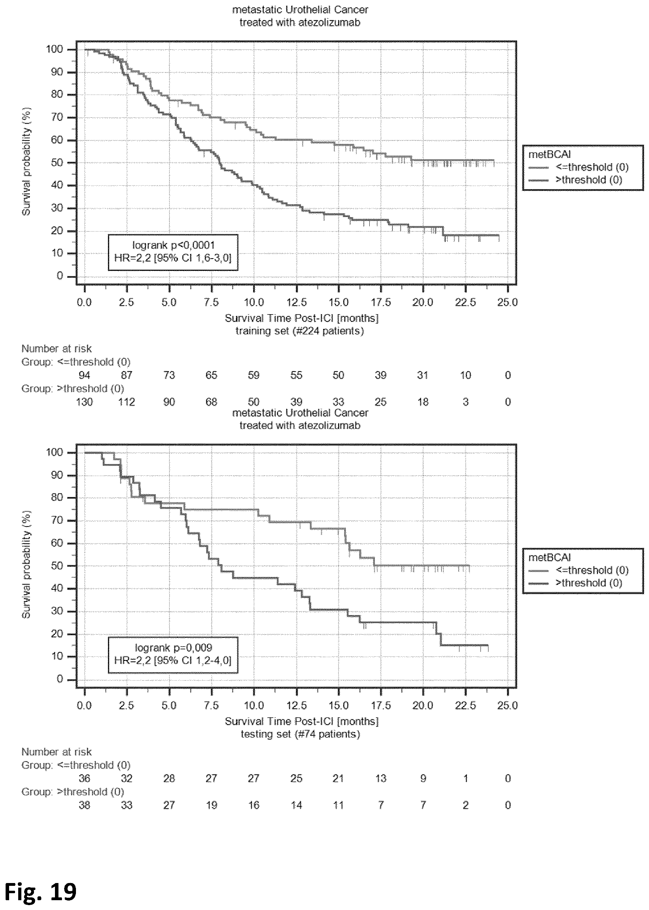

Fig. 19 Kaplan-Meier survival curve analysis of the metastatic BCAI (mBCAI or metBCAI) score in patients with metastatic bladder cancer treated with anti PD-L1 therapy (atezolizumab). The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-3 of the total cohort, top graph) and were fixed for validation in the test cohort (group 4 of the total cohort, bottom graph). In the validation (test) cohort, the mBCAI Score predicts overall survival; patients classified into the high-risk group (>threshold 0) had a 2.2-fold increased hazard risk to die.

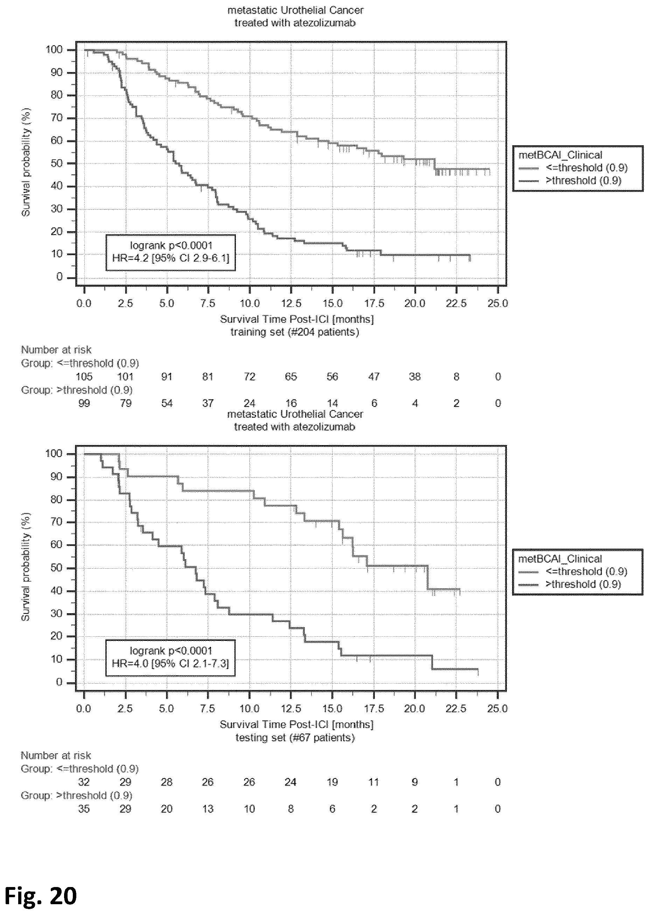

Fig. 20 Kaplan-Meier survival curve analysis of the metastatic BCAI Clinical (mBCAI Clinical or metBCAI Clinical) score in patients with metastatic bladder cancer treated with anti PD-L1 therapy (atezolizumab). The clinical endpoint tested was time to death in months. Patients were stratified into 2 groups according to their risk of experiencing the clinical endpoint as predicted by the respective Cox regression model. The mean value of the prognostic risk scores as derived from the Cox regression was used as a cut-off point for group stratification. Model parameters were established in the train set (groups 1-3 of the total cohort, top graph) and were fixed for validation in the test cohort (group 4 of the total cohort, bottom graph). In the validation (test) cohort, the mBCAI clinical Score predicts overall survival; patients classified into the high-risk group (>threshold 0) had a 4.0-fold increased hazard risk to die.

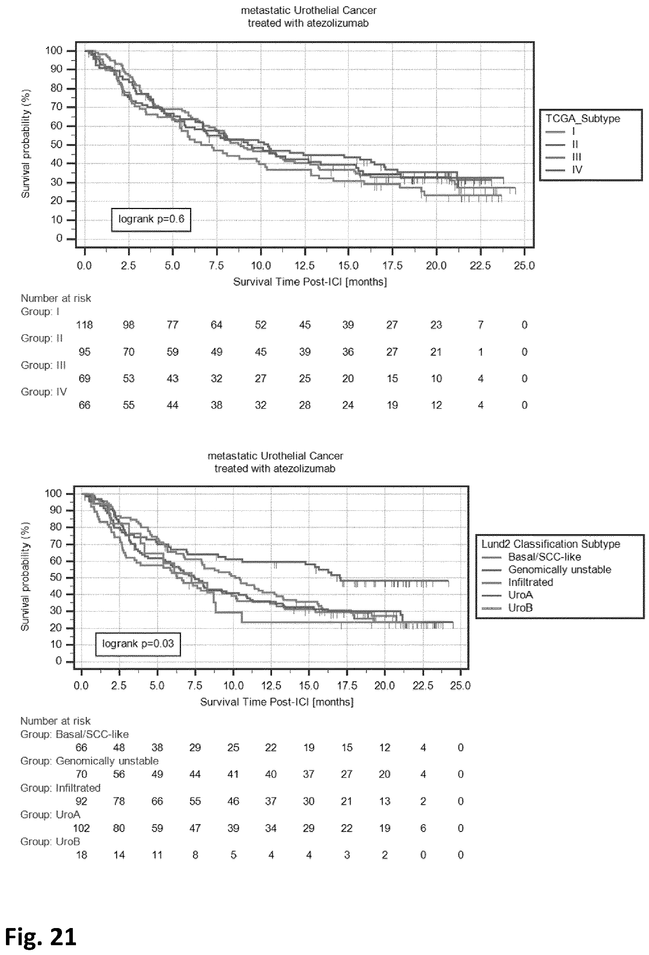

Fig. 21 Kaplan-Meier survival curve analysis of TCGA subtype (top graph) or Lund2 classification (bottom graph) in patients with metastatic bladder cancer treated with anti PD-L1 therapy (atezolizumab). No statistically significant stratification was observed between groups based on the TCGA or Lund2 subtypes.

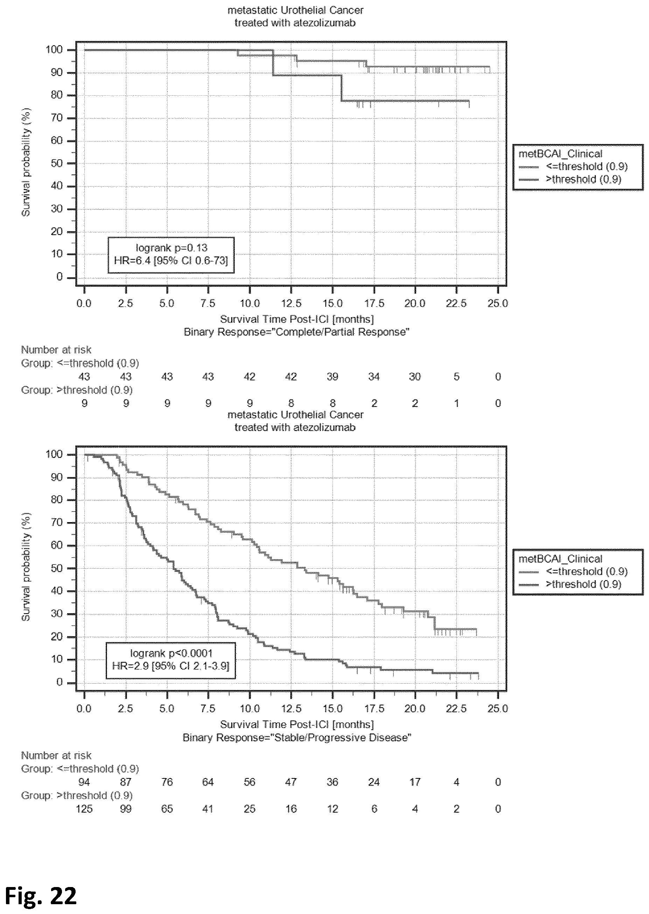

Fig. 22 Patients were divided in anti PD-L1 responders (top graph) and non-responders (bottom graph). Kaplan-Meier plots were generated based on the mBCAI Clinical model. In the non-responder cohort, the mBCAI clinical Score predicts overall survival; patients classified into the high-risk group (>threshold 0) had a 2.9-fold increased hazard risk to die.

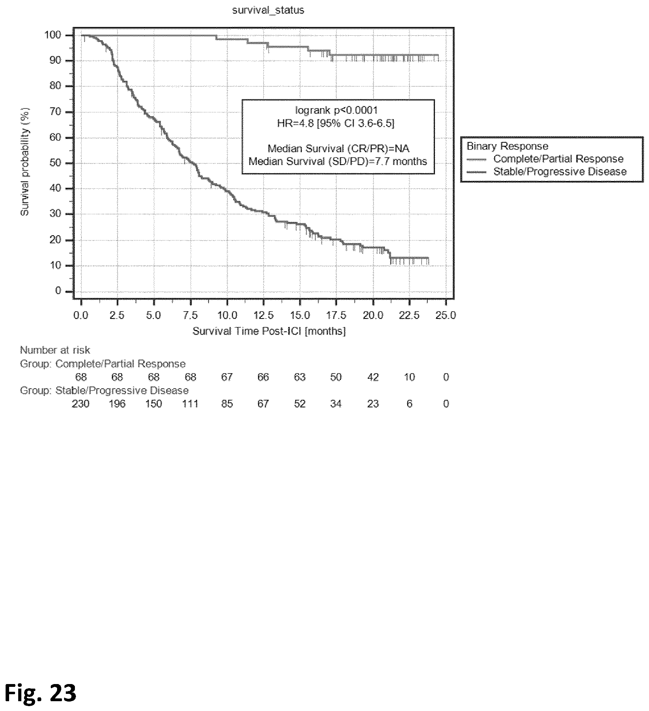

Fig. 23 demonstrates the overall binary response to anti-PD-L1 (atezolizumab) therapy in patients with metastatic bladder cancer. The therapy has a response rate of 22.8%, responders have excellent median survival, while non responders have a median survival of 7.7 months.

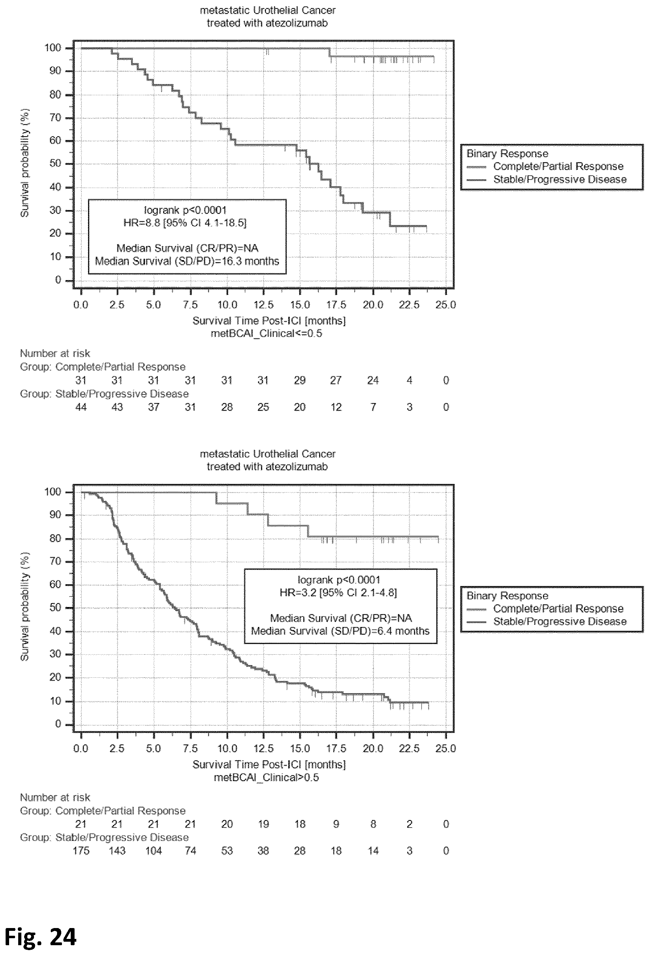

Fig. 24 Patients with metastatic bladder cancer and treated with anti-PD-L1 (atezolizumab) therapy were divided in two groups based on low risk (top graph) or high risk (bottom graph) using the mBCAI score. For each group Kaplan-Meier plots were drafted based on clinical outcome, either Complete Response (CR) / Partial Response (PR) or Progressive Disease (PD) / Stable Disease (SD). The low risk group shows a response rate of 41.3% to anti- PD-L1 therapy and a median survival time of 16.3 months in the non-responders. The high risk group shows a response rate of 10.7% to anti- PD-L1 therapy and a median survival time of months in the non-responders.

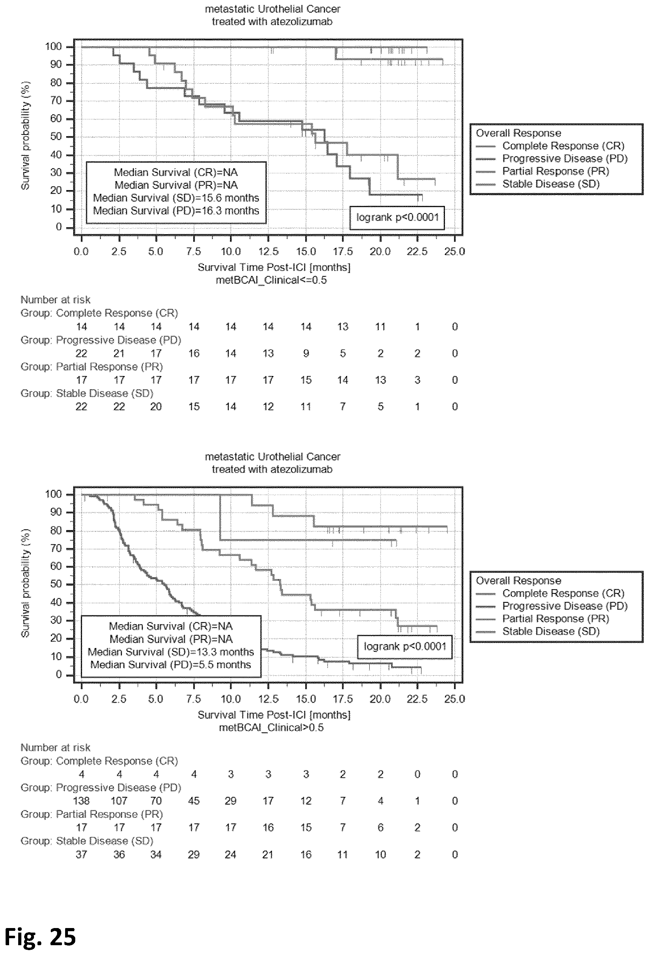

Fig. 25 Patients with metastatic bladder cancer and treated with anti-PD-L1 (atezolizumab) therapy were divided in two groups based on low risk (top graph) or high risk (bottom graph) using the mBCAI score. For each group Kaplan-Meier plots were drafted based on clinical outcome, Complete Response (CR), Partial Response (PR), Progressive Disease (PD) or Stable Disease (SD).

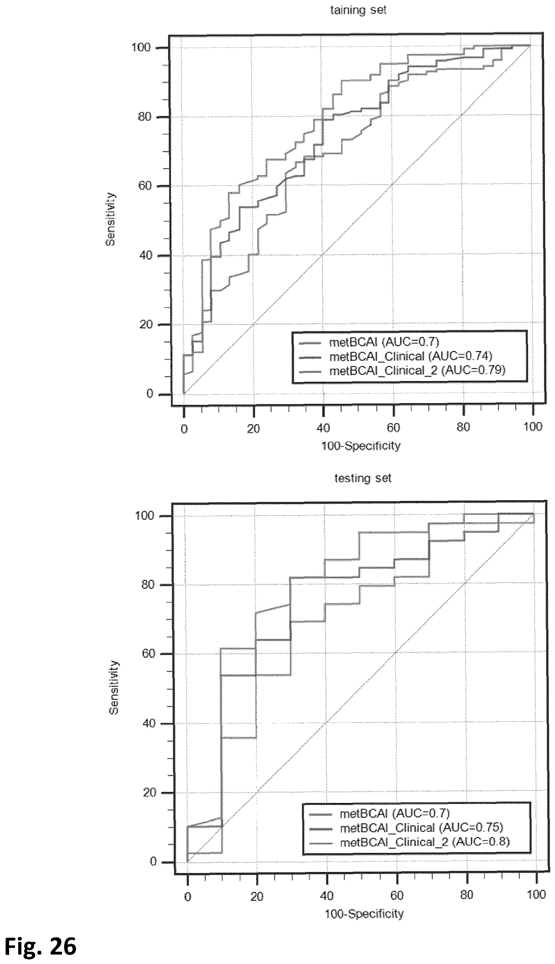

Fig. 26 depicts ROC curves. An AUROC training cohort of 161 patients was used as a training set (top graph) and an AUROC testing cohort of 49 patients was used to validate the model (bottom graph). The clinical endpoint used was the binary response (responder / non-responder to anti-PD-L1 therapy. The plotted models are mBCAI, mBCAI clinical and mBCAI Clinical2. Area under curve (AUC) are indicated for each plot in the inset.

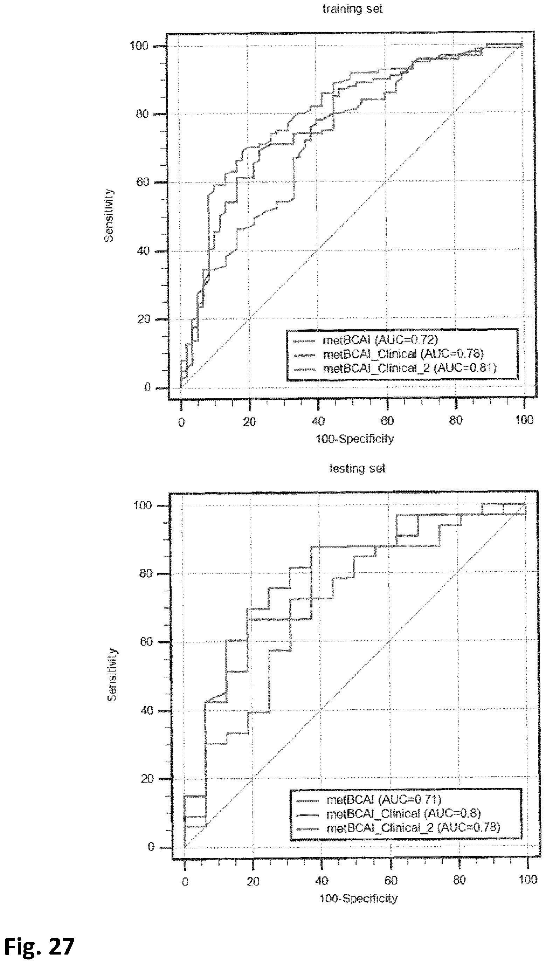

Fig. 27 depicts ROC curves. An AUROC training cohort of 161 patients was used as a training set (top graph) and an AUROC testing cohort of 49 patients was used to validate the model (bottom graph). The clinical endpoint used was overall death. The plotted models are mBCAI, mBCAI clinical

DEFINITIONS

As used herein the indefinite term "a" or "an" does not exclude a plurality.

The term "biological sample" or "sample obtained from a subject" refers to any biological material obtained via suitable methods known to the person skilled in the art from a subject, e.g. a bladder cancer subject.

As used herein, the term "and/or" indicates that one or more of the stated cases may occur, alone or in combination with at least one of the stated cases, up to with all of the stated cases.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

The term "bladder cancer" refers to a cancer of the bladder tissue, which occurs when cells in the bladder mutate and begin to grow out of control.

The term "bladder cancer specific death or disease specific death" refers to death of a patient from a bladder cancer.

The term "clinical recurrence" refers to the presence of clinical signs indicating the presence of tumour cells as measured, for example using in vivo imaging.

As used herein, the word "comprise" or variations thereof such as "comprises" or "comprising" will be understood to include a stated element, integer or step, or group of elements, integers or steps, but not to exclude any other element, integer or steps, or groups of elements, integers or steps. The verb "comprising" includes the verbs "essentially consisting of' and "consisting of".

When used herein, the term "immune defense response genes" is interchangeably used with "IDR genes" or "immune defense genes" and refers to one or more of the genes selected from AIM2, APOBEC3A, CIAO1, DDX58, DHX9, IFI16, IFIH1, IFIT1, IFIT3, LRRFIP1, MYD88, OAS1, TLR8, and ZBP1.

The term "metastases" refers to the presence of metastatic disease in organs other than a bladder tissue.

When used herein, the term "PDE4D7 correlated genes" is interchangeably used with "PDE4D7 genes" and refers to one or more of the genes selected from: ABCC5, CUX2, KIAA1549, PDE4D, RAP1GAP2, SLC39A11, TDRD1, and VWA2.

When used herein, the term "T-cell receptor signalling genes" is interchangeably used with "TCR signalling genes" or "TCR genes" and refers to one or more of the genes selected from: CD2, CD247, CD28, CD3E, CD3G, CD4, CSK, EZR, FYN, LAT, LCK, PAG1, PDE4D, PRKACA, PRKACB, PTPRC, and ZAP70.

DETAILED DESCRIPTION OF THE INVENTION

**Immune system in cancer**

In recent years, the importance of the immune system in cancer inhibition as well as in cancer initiation, promotion and metastasis has become very evident (Mantovani et al. Nature. 454(7203):436-44 (2008); Giraldo et al. Br J Cancer. 120(1):45-53 (2019)). The immune cells and the molecules they secrete form a crucial part of the tumour microenvironment and most immune cells can infiltrate the tumour tissue. The immune system and the tumour affect and shape one another. Thus, antitumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (Giraldo Br J Cancer. 120(1):45-53 (2019)).

While a therapy will be influenced by the immune components of the tumour microenvironment, RT itself extensively affects the make-up of these components. Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is yet uncertain, but its potential for enhancement of cancer immunotherapies is being investigated.

In summary, the status of the immune system and of the immune microenvironment have an impact on therapy effectiveness.

The inventors have identified gene signatures, and combination of these signatures with clinical parameters, of which the resulting models show a significant relation to mortality and therefore are expected to improve the prediction of the effectiveness of these treatments.

**Immune response defense genes**

The integrity and stability of genomic DNA is permanently under stress induced by various cell internal and external factors like exposure to radiation, viral or bacterial infections, but also oxidation and replication stress (see Gasser S. et al. "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g. DNA unnaturally appearing in the cytosolic fraction of the cell in contrast to the nucleus) and damaged DNA (e.g. through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-lbeta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF- kB and interferon responses (light green) are shown).

The factors and mechanisms responsible for activating the DNA sensor pathways in cancer are currently not well elucidated. It will be important to identify the intratumoural DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

**T-Cell receptor signalling genes**

An immune response against pathogens can be elicited at different levels: there are physical barriers, such as the skin, to keep invaders out. If breached, innate immunity comes into play; a first and fast non-specific response. If this is not sufficient, the adaptive immune response is elicited. This is much more specific and needs time to develop when encountering a pathogen for the first time. Lymphocytes are activated by interacting with activated antigen presenting cells from the innate immune system and are also responsible for maintenance of memory for faster responses upon next encounters with the same pathogen.

As lymphocytes are highly specific and effective when activated, they are subject to negative selection for their ability to recognize self, a process known as central tolerance. As not all self-antigens are expressed at selection sites, peripheral tolerance mechanisms evolved as well, such as ligation of the TCR in absence of co-stimulation, expression of inhibitory co-receptors, and suppression by Tregs. A disturbed balance between activation and suppression may lead to autoimmune disorders, or immune deficiencies and cancer, respectively.

T-cell activation can have different functional consequences, depending on the location the type of T-cell involved. CD8+ T-cells differentiate into cytotoxic effector cells, whereas CD4+ T-cells can differentiate into Th1 (IFN$\gamma$ secretion and promotion of cell mediated immunity) or Th2 (IL4/5/13 secretion and promotion of B cell and humoral immunity). Differentiation towards other, more recently identified T-cell subsets is also possible, for example the Tregs, which have a suppressive effect on immune activation (see Mosenden R. and Tasken K., "Cyclic AMP-mediated immune regulation - Overview of mechanisms of action in T-cells", Cell Signal, Vol. 23, No. 6, pages 1009-1016 (2011), in particular, Fig. 4, which T-cell activation and its modulation by PKA, and Tasken K. and Ruppelt A., "Negative regulation of T-cell receptor activation by the cAMP-PKA-Csk signalling pathway in T-cell lipid rafts", Front Biosci, Vol. 11, pages 2929-2939 (2006)).

T cell activation can take place in naive and differentiated T cells. On a molecular level, the events following the ligation of the TCR with cognate antigen, and crosstalk with signalling induced by co-stimulatory and co-inhibitory receptors determine whether the T cell will become activated or if it will become anergic. Just triggering the TCR itself is not enough, this leads to T cell anergy. The B7:CD28 family of co-stimulatory molecules plays a central role in controlling the activation status of T cells upon antigenic stimulation (Torheim E.A., "Immunity Leashed - Mechanisms of Regulation in the Human Immune System", Thesis for the degree of Philosophiae Doctor (PhD), The Biotechnology Centre of Ola, University of Oslo, Norway, 2009).

Activation occurs upon interaction of the TCR on the surface of the T cell with MHC-peptide complexes on APCs or target cells (Figure 2). An immunological synapse is formed, lipid rafts in the T cell membrane coalesce and Lck and Fyn are activated. These molecules phosphorylate ITAMs in the CD3 subunits of the TCR, which facilitates recruitment of Zap-70 in proximation with Lck. Lck phosphorylates and activates Zap-70, which in turn then phosphorylates LAT, SLP76, and PLC$\gamma$1. LAT is a docking site for other signalling molecules and is essential for downstream TCR signalling. Grb2, Gads, PI3K and NCK are recruited to LAT, propagating signalling involving activation of RAS, PKC, mobilization of Ca2+, calcineurin and polymerization of the actin cytoskeleton (Tasken et al. Front in Bioscience 11:2929-2939 (2006)). This eventually leads to activation of transcription factors of the NFkB, NFAT, AP1, and ATF families, resulting in transcription of genes for immune activation (Mosenden et al. Cell Signalling 23:1009-1016 (2011); Tasken et al. Front in Bioscience 11:2929-2939 (2006)).

Both PKA and PDE4 regulated signalling intersect with TCR induced T-cell activation to fine-tune its regulation, with opposing effects (see Abrahamsen H. et al., "TCR- and CD28-mediated recruitment of phosphodiesterase 4 to lipid rafts potentiates TCR signaling", J Immunol, Vol. 173, pages 4847-4848 (2004), in particular, Fig. 6, which shows opposing effects of PKA and PDE4 on TCR activation). The molecule that connects these effectors is cyclic AMP (cAMP), an intracellular second messenger of extracellular ligand action. In T-cells, it mediates effects of prostaglandins, adenosine, histamine, beta-adrenergic agonists, neuropeptide hormones and beta-endorphin. Binding of these extracellular molecules to GPCRs leads to their conformational change, release of stimulatory subunits and subsequent activation of adenylate cyclases (AC), which hydrolyse ATP to cAMP (see Fig. 6 of Abrahamsen H. et al., 2004, ibid). Although not the only one, PKA is the principal effector of cAMP signalling (see Mosenden R. and Tasken K., 2011, ibid, and Tasken K. and Ruppelt A., 2006, ibid). At a functional level, increased levels of cAMP lead to reduced IFN$\gamma$ and IL-2 production in T-cells (see Abrahamsen H. et al., 2004, ibid). Aside from interfering with TCR activation, PKA has many more effectors (see Fig. 15 of Torheim E.A., 2009, ibid).

In naive T-cells, hyperphosphorylated PAG targets Csk to lipid rafts. Via the Ezrin-EBP50-PAG scaffold complex PKA is targeted to Csk. Through specific phosphorylation by PKA, Csk can negatively regulate Lck and Fyn to dampen their activity and downregulate T-cell activation (see Fig. 6 of Abrahamsen H. et al., 2004, ibid). Upon TCR activation, PAG is dephosphorylated and Csk is released from the rafts. Dissociation of Csk is needed for T-cell activation to proceed. Within the same time course, a Csk-G3BP complex is formed and seems to sequester Csk outside lipid rafts (see Mosenden R. and Tasken K., 2011, ibid, and Tasken K. and Ruppelt A., 2006, ibid).

In contrast, combined TCR and CD28 stimulation mediates recruitment of the cyclic nucleotide phosphodiesterase PDE4 to lipid rafts, which enhances cAMP degradation (see Fig. 6 of Abrahamsen H. et al., 2004, ibid). As such, TCR induced production of cAMP is countered, and the T-cell immune response potentiated. Upon TCR stimulation alone, PDE4 recruitment may be too low to fully reduce the cAMP levels and therefore maximal T-cell activation cannot occur (see Abrahamsen H. et al., 2004, ibid).

Thus, by active suppression of proximal TCR signalling, signalling via cAMP-PKA-Csk is thought to set the threshold for T-cell activation. Recruitment of PDEs can counter this suppression. Tissue or cell-type specific regulation is accomplished through expression of multiple isoforms of AC, PKA, and PDEs. As mentioned above, the balance between activation and suppression needs to be tightly regulated to prevent development of autoimmune disorders, immune deficiencies and cancer.

**PDE4D7 correlated genes**

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavoured to describe the complement of PDEs in prostate, all of which identified significant levels of PDE4 expression alongside other PDEs, leading to the development of a PDE4D7 biomarker (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). Since the PDE4D7 biomarker has been proven to be a good predictor, it was assumed that the ability to identify markers that are highly correlated with the PDE47 biomarker might also be helpful in prognosticating the outcome of certain cancer subjects.

Based on the correlation between PDE4D7 expression and pathological features of the disease, the defined aim was to identify prognostic associations between the expression of PDE4D7 in a patient prostate tissue, collected by either biopsy or surgery, and clinically useful information relevant to the outcome of individual patients. Clinically relevant endpoints, or surrogate endpoints that are significantly correlated to the development of metastases, cancer specific or overall mortality have, typically, been evaluated as prognostic cancer biomarkers. The most relevant rational for using a surrogate endpoint relates to situations where either data on established clinical endpoints are not available or when the number of events in the data cohort is too limited for statistical data analysis. For the development of the PDE4D7 prognostic biomarker either BCR (biochemical relapse) progression-free survival or start of post-surgical secondary treatment were evaluated as surrogate endpoints for metastases and prostate cancer death. Using these particular endpoints, a relevant number of events in the selected clinical cohorts (e.g., >30% for BCR) were identified, which is particularly relevant for multivariable data analysis.

In the performed evaluation, standard methods of multivariable analysis such as Cox regression and Kaplan-Meier survival analysis were selected in order to investigate the added and independent value of the continuous and/or the categorical 'PDE4D7 score' compared to established prognostic clinical variables such as PSA and Gleason score (Alves de Inda, 2018). Risk models were built wherein the 'PDE4D7 score' was combined with either pre- or post-surgical clinical predictors of post-surgical progression using logistic regression. The resulting models were subsequently tested on multiple independent patient cohorts in Kaplan-Meier survival and ROC curve analysis in order to predict post-treatment progression free survival (Alves de Inda, 2018).

Using such a strategy, the prognostic value of the PDE4D7 score on a biopsy from retrospectively collected, resected prostate tissue in a consecutively managed patient cohort from a single surgery center in a post-surgical setting (Alves de Inda, 2018) was tested. The patient population comprised some 500 individuals where longitudinal follow-up, of both pathology and biological outcomes, was undertaken. These clinical data were available for all patients and collected during a follow-up of a median 120 months after treatment. The 'PDE4D7 score' was determined as described above and then tested in both uni- and multivariable analyses using the available post-surgical co-variates (i.e. pathology Gleason score, pT stage, surgical margin status, seminal vesicle invasion status, and lymph node invasion status) in order to adjust for the multivariable setting. In this instance, biochemical progression-free survival after primary intervention was set as the evaluated clinical endpoint. The univariable analysis of these clinical samples (Alves de Inda, 2018), showing the inverse association between PDE4D7 expression (in terms of 'PDE4D7 score') and post-surgical biological relapse (HR=0.53 per unit change; 95% CI 0.41-0.67; p<0.0001), robustly confirmed previous data (Boettcher 2015; Boettcher, 2016). In multivariable analysis with such clinical variables, the 'PDE4D7 score' remained as an independent and effective means for predicting clinical outcome (HR=0.56 per unit change; 95% CI 0.43-0.73; p<0.0001). Furthermore, a very similar outcome was obtained when the 'PDE4D7 score' in multivariable analysis (HR=0.54 95% CI 0.42-0.69; p<0.0001) with the validated and clinically-used risk model CAPRA-S was evaluated. The CAPRA-S score, which is based on pre-operative PSA and pathologic parameters determined at the time of surgery, was developed to provide clinicians with information aimed to help predict disease recurrence, including BCR, systemic progression, and PCSM and has been validated in US and other populations.

Interestingly, when assessing the hazard ratio (HR) compared to the continuous 'PDE4D7 score' a linear increase in risk with decreasing 'PDE4D7 score' for score values lying between 2 and 5 was uncovered. However, at PDE4D7 scores less than 2, then the risk of post-surgical progression increases steeply (Alves de Inda, 2018). This is also evident in the Kaplan-Meier survival curves where patients that are grouped within the lowest 'PDE4D7 scores' category exhibit the highest risk of disease recurrence. Using logistic regression analysis, the CAPRA-S score was combined with the continuous 'PDE4D7 score'. Testing this model using ROC curve analysis a 4-6% significant improvement in AUC was noticed when compared to the CAPRA-S alone for both 2- and 5-year predictions of post-treatment progression to BCR. Thus, a combined CAPRA-S & 'PDE4D7 score' Cox regression combination model was evaluated in Kaplan-Meier survival analysis and compared this to the CAPRA-S score categories alone. Undertaking this, it was confirmed that the added value in risk prediction when using a model, the combined 'PDE4D7 & CAPRA-S' score, compared to using the clinical metric of CAPRA-S score alone (Alves de Inda, 2018).

Subsequent to the diagnosis of prostate cancer, an accurate risk assessment needs to be undertaken before stratification to a defined primary treatment. With this in mind, it was tested whether it was possible to translate the prognostic use of the 'PDE4D7 score' in a pre-surgery situation testing tumour tissue obtained from diagnostic needle biopsy samples (van Strijp 2018). In this, needle biopsies were performed on 168 patients, from a single diagnostic clinical centre, who had undergone surgery as a primary treatment. The minimum follow-up period for each patient was 60 months after this intervention. The clinical co-variates used to adjust the 'PDE4D7 score' in the multivariable analysis were age at surgery, pre-operative PSA, PSA density, biopsy Gleason score, percentage of tumour positive biopsy cores, percentage of tumour in the biopsy and clinical cT stage. In this the utility of the 'PDE4D7 score' and the combined 'PDE4D7 & CAPRA' scores compared to the pre-surgical CAPRA score in Cox regression analysis for biochemical relapse (van Strijp 2018) were evaluated.

Evaluating this patient cohort it was found (van Strijp 2018) that the 'PDE4D7 score' was inversely associated with BCR in multivariable analysis when adjusting for clinical variables (HR=0.43; 95% CI 0.29-0.63; p<0.0001) as well as for the clinical CAPRA score (HR=0.53; 95% CI 0.38-0.74; p=0.0001). Kaplan-Meier analysis demonstrated that, as before, in a post-surgical setting, the 'PDE4D7 score' categories were significantly associated with BCR progression free survival (logrank p<0.0001) and secondary treatment free survival (logrank p=0.01). Next a combination logistic regression model, which was developed on the previous cohort, was employed (van Strijp 2018). This consisted of the combined 'CAPRA & PDE4D7' score, demonstrating that patients within the highest combined 'CAPRA & PDE4D7' combined score category have virtually no risk of biochemical progression or transfer to any secondary treatment after surgery. This logistic regression model was also evaluated using ROC curve analysis in order to predict 5-year BCR after surgery. This revealed an increase in AUC of 5% over the CAPRA score alone (AUC=0.82 vs. 0.77, respectively; p=0.004). Decision curve analysis of the combined 'CAPRA & PDE4D7' score model confirmed the superior net benefit of using this combined score, compared to either score alone, across all decision thresholds in order to decide on whether to undertake intervention (e.g. surgery) based on the risk threshold of an individual patient to experience post-surgical disease progression (van Strijp 2018).

The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the treatment response, in order to increase the success rate of these therapies.

**Selection of the genes**

Gene signatures, and combinations of these signatures with clinical parameters, have been identified of which the resulting models show a significant relation to mortality and therefore are expected to improve the prediction of the effectiveness of these treatments.

The identified immune defense response genes AIM2, APOBEC3A, CIAO1, DDX58, DHX9, IFI16, IFIH1, IFIT1, IFIT3, LRRFIP1, MYD88, OAS1, TLR8, and ZBP1, respectively, were identified as follows: A group of 538 prostate cancer patients were treated with RP and the prostate cancer tissue was stored together with clinical (e.g. pathological Gleason grade group (pGGG), pathology state (pT stage)) as well as relevant outcome parameters (e.g. biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis , which resulted in a range of enriched annotation clusters. The annotation cluster #2 demonstrated enrichment (enrichment score: 10.8) in 30 genes with a function in defense response to viruses, negative regulation of viral genome replication as well as type I interferon signalling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signalling was further enriched to 61 genes by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The identified T-Cell receptor signalling genes CD2, CD247, CD28, CD3E, CD3G, CD4, CSK, EZR, FYN, LAT, LCK, PAG1, PDE4D, PRKACA, PRKACB, PTPRC, and ZAP70 were identified as follows: A group of 538 prostate cancer patients were treated with RP and the prostate cancer tissue was stored together with clinical (e.g. pathological Gleason grade group (pGGG), pathology state (pT stage)) as well as relevant outcome parameters (e.g. biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes (see Alves de Inda M. et al., 2018, ibid). PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis, which resulted in a range of enriched annotation clusters. The annotation cluster #6 demonstrated enrichment (enrichment score: 5.9) in 17 genes with a function in primary immune deficiency and activation of T-Cell receptor signalling. A further heat map analysis confirmed that these T-Cell receptor signalling genes were generally higher expressed in samples from patients in PDE4D7 score class 1 than from patients in PDE4D7 score class 4.

The identified PDE4D7 correlated genes ABCC5, CUX2, KIAA1549, PDE4D, RAP1GAP2, SLC39A11, TDRD1, and VWA2 were identified as follows: In RNAseq data generated on 571 prostate cancer patients on close to 60,000 transcripts a range of genes were identified that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. As input data for the calculation of the correlation coefficient the PDE4D7 score (see Alves de Inda M. et al., 2018, ibid) and the RNAseq determined TPM gene expression value per gene of interest (see below) were used.

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. Genes in the range of correlation -0.31 to -0.38 as well as +0.41 to +0.56 were selected. In total 77 transcripts matching these characteristics were identified. From those 77 transcripts the eight PDE4D7 correlated genes ABCC5, CUX2, KIAA1549, PDE4D, RAP1GAP2, SLC39A11, TDRD1, and VWA2 were selected by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT. The boundary condition for the selection of the eight genes was given by the restriction that the p-values in the multivariate Cox-regression were <0.1 for all genes retained in the model.

In this document, it is shown that these genes are also of prognostic value with respect to an outcome of a bladder cancer subject.

Therefore, in a first embodiment the invention provides for a method of predicting an outcome of a bladder cancer subject, comprising: determining or receiving the result of a determination of a gene expression profile comprising three or more, such as 3, 4, 5, 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a favourable outcome or a non-favourable outcome for the subject. Optionally, the method further comprises a step of providing the prediction of the outcome to a medical caregiver or the subject.

In an embodiment the invention provides for a computer implemented method of predicting an outcome of a bladder cancer subject, comprising: receiving the result of a determination of a gene expression profile comprising three or more gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a favourable outcome or a non-favourable outcome for the subject, optionally, providing the prediction of the outcome to a medical caregiver or the subject.

The inventors describe herein that the gene signature may be used to predict survival of a patient diagnosed with bladder cancer. Therefore in a an embodiment the invention provides for a method of predicting an outcome of a bladder cancer subject, comprising: determining or receiving the result of a determination of a gene expression profile comprising three or more, such as 3, 4, 5, 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is survival. The survival may refer to overall survival, or cancer specific death.

The inventors further describe that a treatment response of a bladder cancer patient may be predicted using the gene signature. Therefore in a an embodiment the invention provides for a method of predicting an outcome of a bladder cancer subject, comprising: determining or receiving the result of a determination of a gene expression profile comprising three or more, such as 3, 4, 5, 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a treatment response. The treatment may be surgery or a therapy such as immunotherapy or treatment with an immune checkpoint inhibitor. The immunotherapy may be BCG. The immune checkpoint inhibitor may be anti-PD-L1, for example atezolizumab. The patient may be suffering from NMIBC, MIBC or metastatic bladder cancer.

The present invention describes the use of a gene signature to predict the outcome of a subject with bladder cancer. The gene signature comprises a gene expression profile comprising three or more, such as 3, 4, 5, 6, 7, 8, 9, 10, 11, 12, 13, 14, 16, 17, 18, 19, 20, 21, 22, 23, 24, 25, 26, 27, 28, 29, 30, 31, 32, 33, 34, 35, 36, 37, 38 or even 39, gene expression levels, wherein the three or more gene expression levels are selected from: immune defence response genes and/or T-cell receptor signalling genes and/or PDE4D7 correlated genes. Therefore in an embodiment the three or more genes may be selected from immune defence response genes. In an embodiment the three or more genes may be selected from the T-cell receptor signalling genes. In an embodiment the three or more genes may be selected from the PDE4D7 correlated genes. In an embodiment the three or more genes comprise one or more immune defence response genes and one or more T-cell receptor signalling genes. In an embodiment the three or more genes comprise one or more immune defence response genes and one or more PDE4D7 correlated genes. In an embodiment the three or more genes comprise one or more PDE4D7 correlated genes and one or more T-cell receptor signalling genes. In an embodiment the three or more genes comprise one or more immune defence response genes and one or more T-cell receptor signalling genes and one or more PDE4D7 correlated genes.

With respect to the biological processes described above, three immune system related gene signatures were selected, including the genes listed in tables 1-3. Before, the relevance of these signatures to prediction of prostate cancer survival was shown.

**Table 1:** Immune Defence Response (IDR) Signature.

| Gene Symbol | Ensembl_ID |
|-------------|------------------|
| AIM2 | ENSG00000163568 |
| APOBEC3A | ENSG00000128383 |
| CIAO1 | ENSG00000144021 |
| DDX58 | ENSG00000107201 |
| DHX9 | ENSG00000135829 |
| IFI16 | ENSG00000163565 |
| IFIH1 | ENSG00000115267 |
| IFIT1 | ENSG00000185745 |
| IFIT3 | ENSG00000119917 |
| LRRFIP1 | ENSG00000124831 |
| MYD88 | ENSG00000172936 |
| OAS1 | ENSG00000089127 |
| TLR8 | ENSG00000101916 |
| ZBP1 | ENSG00000124256 |

**Table 2:** T-Cell Receptor (TCR) Signature.

| Gene Symbol | Ensembl_ID |
|-------------|------------------|
| CD2 | ENSG00000116824 |
| CD247 | ENSG00000198821 |
| CD28 | ENSG00000178562 |
| CD3E | ENSG00000198851 |
| CD3G | ENSG00000160654 |
| CD4 | ENSG00000010610 |
| CSK | ENSG00000103653 |
| EZR | ENSG00000092820 |
| FYN | ENSG00000010810 |
| LAT | ENSG00000213658 |
| LCK | ENSG00000182866 |
| PAG1 | ENSG00000076641 |
| PDE4D | ENSG00000113448 |
| PRKACA | ENSG00000072062 |
| PRKACB | ENSG00000142875 |
| PTPRC | ENSG00000081237 |
| ZAP70 | ENSG00000115085 |

**Table 3:** PDE4D7 Correlated (PDE4D7_R2) signature.

| Gene Symbol | Ensembl_ID |
|---|---|
| ABCC5 | ENSG00000114770 |
| CUX2 | ENSG00000111249 |
| KIAA1549 | ENSG00000122778 |
| PDE4D | ENSG00000113448 |
| RAP1GAP2 | ENSG00000132359 |
| SLC39A11 | ENSG00000133195 |
| TDRD1 | ENSG00000095627 |
| VWA2 | ENSG00000165816 |

It was found that the genes AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70, ABCC5, CUX2, KIAA1549, PDE4D, RAP1GAP2, SLC39A11, TDRD1, and VWA2 each individually, one or more per gene panel, in a combination of one or more per gene panel or when all combined, are able to predict the outcome in a bladder cancer subject.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2.

The term "AIM2" refers to the Absent in Melanoma 2 gene (Ensembl: ENSG00000163568), for example, to the sequence as defined in NCBI Reference Sequence NM _004833, specifically, to the nucleotide sequence as set forth in SEQ ID NO:5, which corresponds to the sequence of the above indicated NCBI Reference Sequence of the AIM2 transcript, and also relates to the corresponding amino acid sequence for example as set forth in SEQ ID NO:6, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:7, which corresponds to the sequence of the above indicated NCBI Reference Sequence of the APOBEC3A transcript, and also relates to the corresponding amino acid sequence for example as set forth in SEQ ID NO:8, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g. nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "CD2" refers to the Cluster Of Differentiation 2 gene (Ensembl: ENSG00000116824), for example, to the sequence as defined in NCBI Reference Sequence NM_001767, specifically, to the nucleotide sequence as set forth in SEQ ID NO:9, which corresponds to the sequence of the above indicated NCBI Reference Sequence of the CD2 transcript, and also relates to the corresponding amino acid sequence for example as set forth in SEQ ID NO:10, which corresponds to the protein sequence defined in NCBI Protein Accession Reference Sequence NP_001758 encoding the CD2 polypeptide.

The term "CD2" also comprises nucleotide sequences showing a high degree of homology to CD2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "CD247" refers to the Cluster Of Differentiation 247 gene (Ensembl: ENSG00000198821), for example, to the sequence as defined in NCBI Reference Sequence NM_000734 or in NCBI Reference Sequence NM_198053, specifically, to the nucleotide sequence as set forth in SEQ ID NO:11 or in SEQ ID NO: 12, which correspond to the sequences of the above indicated NCBI Reference Sequences of the CD247 transcript, and also relates to the corresponding amino acid sequence for example as set forth in SEQ ID NO:13 or in SEQ ID NO: 14, which correspond to the protein sequences defined in NCBI Protein Accession Reference Sequence NP_000725 and in NCBI Protein Accession Reference Sequence NP_932170 encoding the CD247 polypeptide.

The term "CD247" also comprises nucleotide sequences showing a high degree of homology to CD247, e.g. nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:13 or in SEQ ID NO:14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO: 12.

The term "CD28" refers to the Cluster Of Differentiation 28 gene (Ensembl: ENSG00000178562), for example, to the sequence as defined in NCBI Reference Sequence NM_006139 or in NCBI Reference Sequence NM_001243078, specifically, to the nucleotide sequence as set forth in SEQ ID NO: 15 or in SEQ ID NO: 16, which correspond to the sequences of the above indicated NCBI Reference Sequences of the CD28 transcript, and also relates to the corresponding amino acid sequence for example as set forth in SEQ ID NO:17 or in SEQ ID NO: 18, which correspond to the protein sequences defined in NCBI Protein Accession Reference Sequence NP_006130 and in NCBI Protein Accession Reference Sequence NP_001230007 encoding the CD28 polypeptide.

The term "CD28" also comprises nucleotide sequences showing a high degree of homology to CD28, e.g. nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16.

The term "CD3E" refers to the Cluster Of Differentiation 3E gene (Ensembl: ENSG00000198851), for example, to the sequence as defined in NCBI Reference Sequence NM_000733, specifically, to the nucleotide sequence as set forth in SEQ ID NO: 19, which corresponds to the sequence of the above indicated NCBI Reference Sequence of the CD3E transcript, and also relates to the corresponding amino acid sequence for example as set forth in SEQ ID NO:20, which corresponds to the protein sequence defined in NCBI Protein Accession Reference Sequence NP_000724 encoding the CD3E polypeptide.

The term "CD3E" also comprises nucleotide sequences showing a high degree of homology to CD3E, e.g. nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 19.

The term "CD3G" refers to the Cluster Of Differentiation 3G gene (Ensembl: ENSG00000160654), for example, to the sequence as defined in NCBI Reference Sequence NM_000073, specifically, to the nucleotide sequence as set forth in SEQ ID NO:21, which corresponds to the sequence of the above indicated NCBI Reference Sequence of the CD3G transcript, and also relates to the corresponding amino acid sequence for example as set forth in SEQ ID NO:22, which corresponds to the protein sequence defined in NCBI Protein Accession Reference Sequence NP_000064 encoding the CD3G polypeptide.

The term "CD3G" also comprises nucleotide sequences showing a high degree of homology to CD3G, e.g. nucleic acid sequences being at least 75%, 80%, 85%, 90%, 91%, 92%, 93%, 94%, 95%, 96%, 97%, 98% or 99% identical to the sequence as set forth in SEQ ID NO:21 or amino acid sequences being at least 75%, 80%, 85%, 90%, 91%, 92%, 93%, 94%, 95%, 96%, 97%, 98% or 99% identical to the sequence as set forth in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21.

The term "CD4" refers to the Cluster Of Differentiation 4 gene (Ensembl: ENSG00000010610), for example, to the sequence as defined in NCBI Reference Sequence NM_000616, specifically, to the nucleotide sequence as set forth in SEQ ID NO:23, which corresponds to the sequence of the above indicated NCBI Reference Sequence of the CD4 transcript, and also relates to the corresponding amino acid sequence for example as set forth in SEQ ID NO:24, which corresponds to the protein sequence defined in NCBI Protein Accession Reference Sequence NP_000607 encoding the CD4 polypeptide.

The term "CD4" also comprises nucleotide sequences showing a high degree of homology to CD4, e.g. nucleic acid sequences being at least 75%, 80%, 85%, 90%, 91%, 92%, 93%, 94%, 95%, 96%, 97%, 98% or 99% identical to the sequence as set forth in SEQ ID NO:23 or amino acid sequences being at least 75%, 80%, 85%, 90%, 91%, 92%, 93%, 94%, 95%, 96%, 97%, 98% or 99% identical to the sequence as set forth in SEQ ID NO:24 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:24 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:23.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:25, which corresponds to the sequence of the above indicated NCBI Reference Sequence of the CIAO1 transcript, and also relates to the corresponding amino acid sequence for example as set forth in SEQ ID NO:26, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:25 or amino acid sequences being at least 75%, 80%, 85%, 90%, 91%, 92%, 93%, 94%, 95%, 96%, 97%, 98% or 99% identical to the sequence as set forth in SEQ ID NO:26 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:26 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:25.

The term "CSK" refers to the C-Terminal Src Kinase gene (Ensembl: ENSG00000103653), for example, to the sequence as defined in NCBI Reference Sequence NM_004383, specifically, to the nucleotide sequence as set forth in SEQ ID NO:27, which corresponds to the sequence of the above indicated NCBI Reference Sequence of the CSK transcript, and also relates to the corresponding amino acid sequence for example as set forth in SEQ ID NO:28, which corresponds to the protein sequence defined in NCBI Protein Accession Reference Sequence NP_004374 encoding the CSK polypeptide.

The term "CSK" also comprises nucleotide sequences showing a high degree of homology to CSK, e.g. nucleic acid sequences being at least 75%, 80%, 85%, 90%, 91%, 92%, 93%, 94%, 95%, 96%, 97%, 98% or 99% identical to the sequence as set forth in SEQ ID NO:27 or amino acid sequences being at least 75%, 80%, 85%, 90%, 91%, 92%, 93%, 94%, 95%, 96%, 97%, 98% or 99% identical to the sequence as set forth in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:27.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:29, which corresponds to the sequence of the above indicated NCBI Reference Sequence of the CUX2 transcript, and also relates to the corresponding amino acid sequence for example as set forth in SEQ ID NO:30, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g. nucleic acid sequences being at least 75%, 80%, 85%, 90%, 91%, 92%, 93%, 94%, 95%, 96%, 97%, 98% or 99% identical to the sequence as set forth in SEQ ID NO:29 or amino acid sequences being at least 75%, 80%, 85%, 90%, 91%, 92%, 93%, 94%, 95%, 96%, 97%, 98% or 99% identical to the sequence as set forth in SEQ ID NO:30 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:30 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:31, which corresponds to the sequence of the above indicated NCBI Reference Sequence of the DDX58 transcript, and also relates to the corresponding amino acid sequence for example as set forth in SEQ ID NO:32, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g. nucleic acid sequences being at least 75%, 80%, 85%, 90%, 91%, 92%, 93%, 94%, 95%, 96%, 97%, 98% or 99% identical to the sequence as set forth in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:31.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:33, which corresponds to the sequence of the above indicated NCBI Reference Sequence of the DHX9 transcript, and also relates to the corresponding amino acid sequence for example as set forth in SEQ ID NO:34, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g. nucleic acid sequences being at least 75%, 80%, 85%, 90%, 91%, 92%, 93%, 94%, 95%, 96%, 97%, 98% or 99% identical to the sequence as set forth in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:33.

The term "EZR" refers to the Ezrin gene (Ensembl: ENSG00000092820), for example, to the sequence as defined in NCBI Reference Sequence NM_003379, specifically, to the nucleotide sequence as set forth in SEQ ID NO:35, which corresponds to the sequence of the above indicated NCBI Reference Sequence of the EZR transcript, and also relates to the corresponding amino acid sequence for example as set forth in SEQ ID NO:36, which corresponds to the protein sequence defined in NCBI Protein Accession Reference Sequence NP_003370 encoding the EZR polypeptide.

The term "EZR" also comprises nucleotide sequences showing a high degree of homology to EZR, e.g. nucleic acid sequences being at least 75%, 80%, 85%, 90%, 91%, 92%, 93%, 94%, 95%, 96%, 97%, 98% or 99% identical to the sequence as set forth in SEQ ID NO:35 or amino acid sequences being at least 75%, 80%, 85%, 90%, 91%, 92%, 93%, 94%, 95%, 96%, 97%, 98% or 99% identical to the sequence as set forth in SEQ ID NO:36 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:36 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35.

The term "FYN" refers to the FYN Proto-Oncogene gene (Ensembl: ENSG00000010810), for example, to the sequence as defined in NCBI Reference Sequence NM_002037 or in NCBI Reference Sequence NM_153047 or in NCBI Reference Sequence NM_153048, specifically, to the nucleotide sequence as set forth in SEQ ID NO:37 or in SEQ ID NO:38 or in SEQ ID NO:39, which correspond to the sequences of the above indicated NCBI Reference Sequences of the FYN transcript, and also relates to the corresponding amino acid sequence for example as set forth in SEQ ID N0:40 or in SEQ ID NO:41 or in SEQ ID NO:42, which correspond to the protein sequences defined in NCBI Protein Accession Reference Sequence NP_002028 and in NCBI Protein Accession Reference Sequence NP_694592 and in NCBI Protein Accession Reference Sequence XP_005266949 encoding the FYN polypeptide.

The term "FYN" also comprises nucleotide sequences showing a high degree of homology to FYN, e.g. nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID N0:40 or in SEQ ID NO:41 or in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID N0:40 or in SEQ ID NO:41 or in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or in SEQ ID NO:39.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:43, which corresponds to the sequence of the above indicated NCBI Reference Sequence of the IFI16 transcript, and also relates to the corresponding amino acid sequence for example as set forth in SEQ ID NO:44, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g. nucleic acid sequences being at least 75%, 80%, 85%, 90%, 91%, 92%, 93%, 94%, 95%, 96%, 97%, 98% or 99% identical to the sequence as set forth in SEQ ID NO:43 or amino acid sequences being at least 75%, 80%, 85%, 90%, 91%, 92%, 93%, 94%, 95%, 96%, 97%, 98% or 99% identical to the sequence as set forth in SEQ ID NO:44 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:44 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:43.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO:45, which corresponds to the sequence of the above indicated NCBI Reference Sequence of the IFIH1 transcript, and also relates to the corresponding amino acid sequence for example as set forth in SEQ ID NO:46, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:45 or amino acid sequences being at least 75%, 80%, 85%, 90%, 91%, 92%, 93%, 94%, 95%, 96%, 97%, 98% or 99% identical to the sequence as set forth in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:45.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO:47 or in SEQ ID NO:48, which correspond to the sequences of the above indicated NCBI Reference Sequences of the IFIT1 transcript, and also relates to the corresponding amino acid sequence for example as set forth in SEQ ID NO:49 or in SEQ ID NO:50, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:51, which corresponds to the sequence of the above indicated NCBI Reference Sequence of the IFIT3 transcript, and also relates to the corresponding amino acid sequence for example as set forth in SEQ ID NO:52, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g. nucleic acid sequences being at least 75%, 80%, 85%, 90%, 91%, 92%, 93%, 94%, 95%, 96%, 97%, 98% or 99% identical to the sequence as set forth in SEQ ID NO:51 or amino acid sequences being at least 75%, 80%, 85%, 90%, 91%, 92%, 93%, 94%, 95%, 96%, 97%, 98% or 99% identical to the sequence as set forth in SEQ ID NO:52 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:52 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:53 or in SEQ ID NO:54, which correspond to the sequences of the above indicated NCBI Reference Sequence of the KIAA1549 transcript, and also relates to the corresponding amino acid sequence for example as set forth in SEQ ID NO:55 or in SEQ ID NO:56, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g. nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54 or amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:53 or in SEQ ID NO:54.

The term "LAT" refers to the Linker For Activation Of T-Cells gene (Ensembl: ENSG00000213658), for example, to the sequence as defined in NCBI Reference Sequence NM_001014987 or in NCBI Reference Sequence NM_014387, specifically, to the nucleotide sequence as set forth in SEQ ID NO:57 or in SEQ ID NO:58, which correspond to the sequences of the above indicated NCBI Reference Sequences of the LAT transcript, and also relates to the corresponding amino acid sequence for example as set forth in SEQ ID NO:59 or in SEQ ID NO:60, which corresponds to the protein sequences defined in NCBI Protein Accession Reference Sequence NP_001014987 and in NCBI Protein Accession Reference Sequence NP_055202 encoding the LAT polypeptide.

The term "LAT" also comprises nucleotide sequences showing a high degree of homology to LAT, e.g. nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58 or amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:59 or in SEQ ID NO:60 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:57 or in SEQ ID NO:58.

The term "LCK" refers to the LCK Proto-Oncogene gene (Ensembl: ENSG00000182866), for example, to the sequence as defined in NCBI Reference Sequence NM_005356, specifically, to the nucleotide sequence as set forth in SEQ ID NO:61, which corresponds to the sequence of the above indicated NCBI Reference Sequence of the LCK transcript, and also relates to the corresponding amino acid sequence for example as set forth in SEQ ID NO:62, which corresponds to the protein sequence defined in NCBI Protein Accession Reference Sequence NP_005347 encoding the LCK polypeptide.

The term "LCK" also comprises nucleotide sequences showing a high degree of homology to LCK, e.g. nucleic acid sequences being at least 75%, 80%, 85%, 90%, 91%, 92%, 93%, 94%, 95%, 96%, 97%, 98% or 99% identical to the sequence as set forth in SEQ ID NO:61 or amino acid sequences being at least 75%, 80%, 85%, 90%, 91%, 92%, 93%, 94%, 95%, 96%, 97%, 98% or 99% identical to the sequence as set forth in SEQ ID NO:62 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:62 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:61.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:63 or in SEQ ID NO:64 or in SEQ ID NO:65 or in SEQ ID NO:66, which correspond to the sequences of the above indicated NCBI Reference Sequences of the LRRFIP1 transcript, and also relates to the corresponding amino acid sequence for example as set forth in SEQ ID NO:67 or in SEQ ID NO:68 or in SEQ ID NO:69 or in SEQ ID NO:70, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66 or amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:67 or in SEQ ID NO:68 or in SEQ ID NO:69 or in SEQ ID NO:70 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:63 or in SEQ ID NO:64 or in SEQ ID NO:65 or in SEQ ID NO:66.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:71 or in SEQ ID NO:72 or in SEQ ID NO:73 or in SEQ ID NO:74 or in SEQ ID NO:75, which correspond to the sequences of the above indicated NCBI Reference Sequences of the MYD88 transcript, and also relates to the corresponding amino acid sequence for example as set forth in SEQ ID NO:76 or in SEQ ID NO:77 or in SEQ ID NO:78 or in SEQ ID NO:79 or in SEQ ID NO:80, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g. nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75 or amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:76 or in SEQ ID NO:77 or in SEQ ID NO:78 or in SEQ ID NO:79 or in SEQ ID NO:80 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:71 or in SEQ ID NO:72 or in SEQ ID NO:73 or in SEQ ID NO:74 or in SEQ ID NO:75.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:81 or in SEQ ID NO:82 or in SEQ ID NO:83 or in SEQ ID NO:84, which correspond to the sequences of the above indicated NCBI Reference Sequences of the OAS1 transcript, and also relates to the corresponding amino acid sequences for example as set forth in SEQ ID NO:85 or in SEQ ID NO:86 or in SEQ ID NO:87 or in SEQ ID NO:88, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84 or amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:85 or in SEQ ID NO:86 or in SEQ ID NO:87 or in SEQ ID NO:88 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:81 or in SEQ ID NO:82 or in SEQ ID NO:83 or in SEQ ID NO:84.

The term "PAG1" refers to the Phosphoprotein Membrane Anchor With Glycosphingolipid Microdomains 1 gene (Ensembl: ENSG00000076641), for example, to the sequence as defined in NCBI Reference Sequence NM_018440, specifically, to the nucleotide sequence as set forth in SEQ ID NO:89, which corresponds to the sequence of the above indicated NCBI Reference Sequence of the PAG1 transcript, and also relates to the corresponding amino acid sequence for example as set forth in SEQ ID NO:90, which corresponds to the protein sequence defined in NCBI Protein Accession Reference Sequence NP_060910 encoding the PAG1 polypeptide.

The term "PAG1" also comprises nucleotide sequences showing a high degree of homology to PAG1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:89 or amino acid sequences being at least 75%, 80%, 85%, 90%, 91%, 92%, 93%, 94%, 95%, 96%, 97%, 98% or 99% identical to the sequence as set forth in SEQ ID NO:90 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:90 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:89.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99, which correspond to the sequences of the above indicated NCBI Reference Sequence of the PDE4D transcript, and also relates to the corresponding amino acid sequence for example as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g. nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99 or amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:100 or in SEQ ID NO:101 or in SEQ ID NO:102 or in SEQ ID NO:103 or in SEQ ID NO:104 or in SEQ ID NO:105 or in SEQ ID NO:106 or in SEQ ID NO:107 or in SEQ ID NO:108 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:91 or in SEQ ID NO:92 or in SEQ ID NO:93 or in SEQ ID NO:94 or in SEQ ID NO:95 or in SEQ ID NO:96 or in SEQ ID NO:97 or in SEQ ID NO:98 or in SEQ ID NO:99.

The term "PRKACA" refers to the Protein Kinase cAMP-Activated Catalytic Subunit Alpha gene (Ensembl:

ENSG00000072062), for example, to the sequence as defined in NCBI Reference Sequence NM_002730 or in NCBI Reference Sequence NM_207518, specifically, to the nucleotide sequences as set forth in SEQ ID NO:109 or in SEQ ID NO:110, which correspond to the sequences of the above indicated NCBI Reference Sequences of the PRKACA transcript, and also relates to the corresponding amino acid sequence for example as set forth in SEQ ID NO:111 or in SEQ ID NO:112, which correspond to the protein sequences defined in NCBI Protein Accession Reference Sequence NP_002721 and in NCBI Protein Accession Reference Sequence NP_997401 encoding the PRKACA polypeptide.

The term "PRKACA" also comprises nucleotide sequences showing a high degree of homology to PRKACA, e.g. nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110 or amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:111 or in SEQ ID NO:112 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:109 or in SEQ ID NO:110.

The term "PRKACB" refers to the Protein Kinase cAMP-Activated Catalytic Subunit Beta gene (Ensembl: ENSG00000142875), for example, to the sequence as defined in NCBI Reference Sequence NM_002731 or in NCBI Reference Sequence NM_182948 or in NCBI Reference Sequence NM_001242860 or in NCBI Reference Sequence NM_001242859 or in NCBI Reference Sequence NM_001242858 or in NCBI Reference Sequence NM_001242862 or in NCBI Reference Sequence NM_001242861 or in NCBI Reference Sequence NM_001300915 or in NCBI Reference Sequence NM_207578 or in NCBI Reference Sequence NM_001242857 or in NCBI Reference Sequence NM_001300917, specifically, to the nucleotide sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123, which correspond to the sequences of the above indicated NCBI Reference Sequences of the PRKACB transcript, and also relates to the corresponding amino acid sequence for example as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134, which correspond to the protein sequences defined in NCBI Protein Accession Reference Sequence NP_002722 and in NCBI Protein Accession Reference Sequence NP_891993 and in NCBI Protein Accession Reference Sequence NP_001229789 and in NCBI Protein Accession Reference Sequence NP_001229788 and in NCBI Protein Accession Reference Sequence NP_001229787 and in NCBI Protein Accession Reference Sequence NP_001229791 and in NCBI Protein Accession Reference Sequence NP_001229790 and in NCBI Protein Accession Reference Sequence NP_001287844 and in NCBI Protein Accession Reference Sequence NP_997461 and in NCBI Protein Accession Reference Sequence NP_001229786 and in NCBI Protein Accession Reference Sequence NP_001287846 encoding the PRKACB polypeptide.

The term "PRKACB" also comprises nucleotide sequences showing a high degree of homology to PRKACB, e.g. nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123 or amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:124 or in SEQ ID NO:125 or in SEQ ID NO:126 or in SEQ ID NO:127 or in SEQ ID NO:128 or in SEQ ID NO:129 or in SEQ ID NO:130 or in SEQ ID NO:131 or in SEQ ID NO:132 or in SEQ ID NO:133 or in SEQ ID NO:134 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:113 or in SEQ ID NO:114 or in SEQ ID NO:115 or in SEQ ID NO:116 or in SEQ ID NO:117 or in SEQ ID NO:118 or in SEQ ID NO:119 or in SEQ ID NO:120 or in SEQ ID NO:121 or in SEQ ID NO:122 or in SEQ ID NO:123.

The term "PTPRC" refers to the Protein Tyrosine Phosphatase Receptor Type C gene (Ensembl: ENSG00000081237), for example, to the sequence as defined in NCBI Reference Sequence NM_002838 or in NCBI Reference Sequence NM_080921, specifically, to the nucleotide sequence as set forth in SEQ ID NO:135 or in SEQ ID NO:136, which correspond to the sequences of the above indicated NCBI Reference Sequences of the PTPRC transcript, and also relates to the corresponding amino acid sequence for example as set forth in SEQ ID NO:137 or in SEQ ID NO:138, which correspond to the protein sequences defined in NCBI Protein Accession Reference Sequence NP_002829 encoding the PTPRC polypeptide and in NCBI Protein Accession Reference Sequence NP_563578.

The term "PTPRC" also comprises nucleotide sequences showing a high degree of homology to PTPRC, e.g.

nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136 or amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:137 or in SEQ ID NO:138 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:135 or in SEQ ID NO:136.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:139 or in SEQ ID NO:140 or in SEQ ID NO:141, which correspond to the sequences of the above indicated NCBI Reference Sequences of the RAP1GAP2 transcript, and also relates to the corresponding amino acid sequence for example as set forth in SEQ ID NO:142 or in SEQ ID NO:143 or in SEQ ID NO:144, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the RAP1GAP2 polypeptide.

The term "RAP1GAP2" also comprises nucleotide sequences showing a high degree of homology to RAP1GAP2, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO:140 or in SEQ ID NO:141 or amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO:143 or in SEQ ID NO:144 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 142 or in SEQ ID NO:143 or in SEQ ID NO: 144 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 139 or in SEQ ID NO: 140 or in SEQ ID NO: 141.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:145 or in SEQ ID NO:146, which correspond to the sequences of the above indicated NCBI Reference Sequences of the SLC39A11 transcript, and also relates to the corresponding amino acid sequence for example as set forth in SEQ ID NO:147 or in SEQ ID NO:148, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 145 or in SEQ ID NO:146 or amino acid sequences being at least 75%, 80%, 85%, 90%, 91%, 92%, 93%, 94%, 95%, 96%, 97%, 98% or 99% identical to the sequence as set forth in SEQ ID NO:147 or in SEQ ID NO: 148 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 147 or in SEQ ID NO: 148 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:145 or in SEQ ID NO: 146.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO: 149, which corresponds to the sequence of the above indicated NCBI Reference Sequence of the TDRD1 transcript, and also relates to the corresponding amino acid sequence for example as set forth in SEQ ID NO: 150, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 149 or amino acid sequences being at least 75%, 80%, 85%, 90%, 91%, 92%, 93%, 94%, 95%, 96%, 97%, 98% or 99% identical to the sequence as set forth in SEQ ID NO: 150 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 150 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 149.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:151 or in SEQ ID NO:152, which correspond to the sequences of the above indicated NCBI Reference Sequences of the TLR8 transcript, and also relates to the corresponding amino acid sequences for example as set forth in SEQ ID NO:153 or in SEQ ID NO: 154, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g. nucleic acid sequences being at least 75%, 80%, 85%, 90%, 91%, 92%, 93%, 94%, 95%, 96%, 97%, 98% or 99% identical to the sequence as set forth in SEQ ID NO:151 or in SEQ ID NO:152 or amino acid sequences being at least 75%, 80%, 85%, 90%, 91%, 92%, 93%, 94%, 95%, 96%, 97%, 98% or 99% identical to the sequence as set forth in SEQ ID NO:153 or in SEQ ID NO:154 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 153 or in SEQ ID NO: 154 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 151 or in SEQ ID NO: 152.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:155, which corresponds to the sequence of the above indicated NCBI Reference Sequence of the VWA2 transcript, and also relates to the corresponding amino acid sequence for example as set forth in SEQ ID NO:156, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g. nucleic acid sequences being at least 75%, 80%, 85%, 90%, 91%, 92%, 93%, 94%, 95%, 96%, 97%, 98% or 99% identical to the sequence as set forth in SEQ ID NO: 155 or amino acid sequences being at least 75%, 80%, 85%, 90%, 91%, 92%, 93%, 94%, 95%, 96%, 97%, 98% or 99% identical to the sequence as set forth in SEQ ID NO:156 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 156 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 155.

The term "ZAP70" refers to the Zeta Chain Of T-Cell Receptor Associated Protein Kinase 70 gene (Ensembl: ENSG00000115085), for example, to the sequence as defined in NCBI Reference Sequence NM_001079 or in NCBI Reference Sequence NM_207519, specifically, to the nucleotide sequences as set forth in SEQ ID NO:157 or in SEQ ID NO:158, which correspond to the sequences of the above indicated NCBI Reference Sequences of the ZAP70 transcript, and also relates to the corresponding amino acid sequence for example as set forth in SEQ ID NO:159 or in SEQ ID NO:160, which corresponds to the protein sequences defined in NCBI Protein Accession Reference Sequence NP_001070 and in NCBI Protein Accession Reference Sequence NP_997402 encoding the ZAP70 polypeptide.

The term "ZAP70" also comprises nucleotide sequences showing a high degree of homology to ZAP70, e.g. nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158or amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:159 or in SEQ ID NO:160 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:157 or in SEQ ID NO:158.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:161 or in SEQ ID NO:162 or in SEQ ID NO:163, which corresponds to the sequence of the above indicated NCBI Reference Sequence of the ZBP1 transcript, and also relates to the corresponding amino acid sequence for example as set forth in SEQ ID NO:164 or in SEQ ID NO:165 or in SEQ ID NO:166, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g. nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163 or amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:164 or in SEQ ID NO:165 or in SEQ ID NO:166 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:161 or in SEQ ID NO:162 or in SEQ ID NO:163.

As can be derived from figures 4 to 6, each of the individual sub-signatures, meaning either the Immune defense set of genes, the T-cell signaling set of genes or the PDE4D7 related genes may be used to predict overall survival. Therefore it is deemed plausible that a prediction (e.g. for survival or a treatment response) can be made based on three or more genes from a single sub-signature. Therefore in an embodiment, the invention relates to a method of predicting an outcome of a bladder cancer subject, comprising: determining or receiving the result of a determination of a gene expression profile comprising three or more gene expression levels, for example 3, 4, 5, 6, 7, 8, 9, 10, 11, 12, 13, or even 14 expression levels, wherein the three or more, for example 3, 4, 5, 6, 7, 8, 9, 10, 11, 12, 13, or even 14 expression levels, gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profile being determined in a biological sample obtained from the subject, determining the prediction of the outcome based on the gene expression profile, wherein said prediction is a favourable outcome or a non-favourable outcome for the subject, optionally, providing the prediction of the outcome to a medical caregiver or the subject.

Alternatively, the invention relates to a method of predicting an outcome of a bladder cancer subject, comprising: determining or receiving the result of a determination of a gene expression profile comprising three or more gene expression levels, for example 3, 4, 5, 6, 7, 8, 9, 10, 11, 12, 13, 14, 15, 16 or even 17 expression levels, wherein the three or more, for example 3, 4, 5, 6, 7, 8, 9, 10, 11, 12, 13, 14, 15, 16 or even 17 expression levels, gene expression levels are selected from: T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, said gene expression profile being determined in a biological sample obtained from the subject, determining the prediction of the outcome based on the gene expression profile, wherein said prediction is a favourable outcome or a non-favourable outcome for the subject, optionally, providing the prediction of the outcome to a medical caregiver or the subject.

Alternatively, the invention relates to a method of predicting an outcome of a bladder cancer subject, comprising: determining or receiving the result of a determination of a gene expression profile comprising three or more gene expression levels, for example 3, 4, 5, 6, 7, or even 8 expression levels, wherein the three or more, for example 3, 4, 5, 6, 7, or even 8 expression levels, gene expression levels are selected from: PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject, determining the prediction of the outcome based on the gene expression profile, wherein said prediction is a favourable outcome or a non-favourable outcome for the subject, optionally, providing the prediction of the outcome to a medical caregiver or the subject.

In a preferred embodiment, the gene expression profile comprises at least one gene from each of the immune defence response genes, the T-Cell receptor signalling genes and the PDE4D7 correlated genes. Therefore, in an embodiment the three or more genes comprise one or more, e.g. one, two three, four, five six or more, immune defence response gene, one or more, e.g. one, two three, four, five six or more, T-Cell receptor signalling gene and one or more, e.g. one, two three, four, five six or more, PDE4D7 correlated gene. For example the three or more genes comprise at least one immune defence response gene, T-Cell receptor signalling gene and PDE4D7 correlated gene, or the three or more genes comprise at least two immune defence response genes, T-Cell receptor signalling genes and PDE4D7 correlated genes, or the three or more genes comprise at least three immune defence response genes, T-Cell receptor signalling genes and PDE4D7 correlated genes. In an embodiment the one or more immune defense response genes comprise three or more, preferably, six or more, more preferably, nine or more, most preferably, all of the immune defense genes, and/or the one or more T-Cell receptor signalling genes comprise three or more, preferably, six or more, more preferably, nine or more, most preferably, all of the T-Cell receptor signalling genes, and/or the one or more PDE4D7 correlated genes comprise three or more, preferably, six or more, most preferably, all of the PDE4D7 correlated genes. In ab embodiment the determining of the prediction of the outcome comprises combining the three or more gene expression levels with a regression function that has been derived from a population of bladder cancer subjects.

As provided herein the biological sample used may be collected in a clinically acceptable manner, e.g. in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a tissue, for example a glandular tissue, e.g. the sample may be derived from the bladder of a subject. Additionally, cells may be purified from obtained body tissues and fluids if necessary, and then used as the biological sample. In some realizations, the sample may be a tissue sample, a fluid sample, a blood sample, a saliva sample, a sample including circulating tumour cells, extracellular vesicles, a sample containing bladder secreted exosomes, or cell lines or cancer cell line. In one particular realization, biopsy or resections samples may be obtained and/or used. Such samples may include cells or cell lysates.

Therefore, in an embodiment the biological sample obtained from a subject is a biopsy. In a further preferred embodiment, the method includes providing or obtaining a biopsy. In a preferred embodiment the biopsy is a bladder biopsy, for example a tissue or a fluid from the bladder.

It is also conceivable that the content of a biological sample is submitted to an enrichment step. For instance, a sample may be contacted with ligands specific for the cell membrane or organelles of certain cell types, e.g. bladder cells, functionalized for example with magnetic particles. The material concentrated by the magnetic particles may subsequently be used for detection and analysis steps as described herein above or below.

Furthermore, cells, e.g. tumour cells, may be enriched via filtration processes of fluid or liquid samples, e.g. blood, urine, etc. Such filtration processes may also be combined with enrichment steps based on ligand specific interactions as described herein above.

It is preferred that a biological sample as provided herein is obtained from the subject before the start of the therapy. It is also preferred that said biological sample is a bladder sample or a bladder cancer sample.

Thus, in a preferred embodiment the method according to the invention comprises that the biological sample is obtained from the subject before the start of the therapy, preferably wherein the biological sample is a bladder sample or a bladder cancer sample. Alternatively, the method according to the invention comprises providing the biological sample obtained from the subject before the start of the therapy, preferably wherein the biological sample is a bladder sample or a bladder cancer sample.

It is contemplated herein that the outcome of a bladder cancer subject may be a favourable one or a non-favourable one. In one aspect of the invention a prediction of the outcome of a bladder cancer subject can result in the determination of a favourable risk or a non-favourable risk of a certain one or more outcomes. The outcomes may comprise overall death or overall survival, bladder-cancer related death, loco-regional recurrence and/or distant recurrence. Preferably, bladder-cancer related death is bladder-cancer specific death. A prediction provided by the method of the invention can provide for a prediction of the risk of a bladder cancer subject for a certain outcome. Moreover, the method of the invention can predict whether the subject having a bladder cancer has a low risk of a certain outcome or a high risk of a certain outcome. The outcomes bladder-cancer related death, loco-regional recurrence and/or distant recurrence, as used herein, comprise outcomes that are non-favourable to a subject. A further outcome is overall death. Overall death as used herein is an outcome that can be predicted by the method of the invention, but that is not directly associable with death of a subject due to the bladder cancer. Therefore in an embodiment the method provides for a prediction of a favourable or a non-favourable outcome, wherein said favourable outcome or a non-favourable outcome is a probability of survival, overall survival, cancer free survival, overall death, or cancer specific death.

It is preferred that the method of the invention as provided comprises that the predicting of an outcome of a bladder cancer subject comprises that the prediction is a favourable risk or a non-favourable risk of bladder-cancer related death, loco-regional recurrence and/or distant recurrence after surgery. In other words, surgery is performed on a bladder cancer subject prior to predicting the risk of a certain outcome. Additionally, the method of the invention as provided comprises that the predicting of an outcome of a bladder cancer subject comprises that the prediction is a favourable risk or a non-favourable risk of bladder-cancer related death, loco-regional recurrence and/or distant recurrence after treatment. For example the treatment may be immunotherapy such as BCG therapy or treatment with an immune checkpoint inhibitor. Therefore in an embodiment the method provides for a prediction of a favourable or a non-favourable outcome, wherein said favourable outcome or non-favourable outcome is survival in response to a treatment. In a further embodiment the treatment is surgery or immunotherapy, preferably wherein the immunotherapy is Bacillus Calmette-Guerin (BCG) immunotherapy or immune checkpoint inhibitor therapy.

A bladder cancer subject having a high risk (i.e. above a certain threshold) to one or more of the outcomes bladder-cancer related death, loco-regional recurrence and/or distant recurrence, as predicted by the method of the invention, is contemplated to be associated with a non-favourable risk, preferably after surgery, for a respective outcome.

A bladder cancer subject having a low risk (i.e. below or equal to a certain threshold) to one or more of the outcomes bladder-cancer related death, loco-regional recurrence and/or distant recurrence, as predicted by the method of the invention, is contemplated to be associated with a favourable risk, preferably after surgery, for a respective outcome. A favourable risk may comprise a different follow-up strategy, for example a different recommended (follow-up) therapy, than a non-favourable risk for a subject. For example, a different follow-up strategy, for example a different recommended (follow-up) therapy, can be considered for a bladder cancer subject, preferably who has undergone surgery of the bladder (cancer), to improve survival chances for said bladder cancer subject.

It is preferred that a therapy comprises surgery, radiotherapy, hormone therapy, cytotoxic chemotherapy and/or immunotherapy. Combination therapy in cancer, i.e. a therapy that combines two or more therapeutic methods and/or agents, for example combining radiotherapy and chemotherapy, is widely regarded as a cornerstone in treating cancer.

Thus, in a preferred embodiment the method according to the invention comprises that the biological sample, preferably the sample of a bladder of a subject or bladder cancer of a subject, is obtained prior to the start of therapy comprising surgery, radiotherapy, hormone therapy, cytotoxic chemotherapy and/or immunotherapy.

A non-favourable risk of an outcome is contemplated to impact the recommended therapy of said bladder cancer subject associated with the non-favourable risk. In a case wherein a non-favourable risk is predicted by the method of the invention it is contemplated that the recommended therapy comprises one or more of:

(i) radiotherapy provided earlier than is the standard; and
(ii) radiotherapy with an increased radiation dose; and
(iii) an adjuvant therapy, such as cytotoxic chemotherapy, immunotherapy and/or hormone therapy; and
(iv) surgery; and
(v) an alternative therapy that is not surgery.

In a preferred embodiment the method according to the invention comprises that a therapy is recommended based on the prediction, preferably the prediction on the outcome, wherein:

- if the prediction is non-favourable, the recommended therapy, preferably after surgery, comprises one or more of:

(i) radiotherapy provided earlier than is the standard; and
(ii) radiotherapy with an increased radiation dose; and
(iii) an adjuvant therapy, such as cytotoxic chemotherapy, immunotherapy and/or hormone therapy; and
(iv) surgery; and
(v) an alternative therapy that is not surgery.

A favourable risk of an outcome is contemplated to impact the recommended therapy of said bladder cancer subject associated with the favourable risk. In a case wherein a favourable risk is predicted by the method of the invention it is contemplated that the recommended therapy comprises one or more of:

(vi) radical radiotherapy; and
(vii) salvage radiotherapy; and
(viii) salvage radiotherapy at de-escalated dose levels; and
(ix) watchful waiting.

Thus, another preferred embodiment of the method according to the invention comprises that a therapy is recommended based on the prediction, wherein:

- if the prediction is favourable, the recommended therapy, preferably after surgery, comprises one or more of:

(vi) radical radiotherapy; and
(vii) salvage radiotherapy; and
(viii) salvage radiotherapy at de-escalated dose levels; and
(ix) watchful waiting.

In one embodiment of the invention the favourable risk or non-favourable risk of an outcome is predicted for a bladder cancer subject that has undergone surgery, preferably surgery on the bladder such as, but not limited to, lumpectomy, quadrantectomy, partial mastectomy, segmental mastectomy or complete mastectomy.
In another aspect of the invention is provided a method in accordance with the invention comprising that secondary treatment is recommended for a certain patient, preferably for a patient having a low or a high risk of suffering an outcome selected from bladder-cancer specific death or overall death, wherein the recommendation is based on the prediction of the outcome of a bladder cancer patient, wherein:

- if the prediction is favourable no secondary treatment is recommended; and/or
- if the prediction is non-favourable secondary treatment is recommended.

It is provided herein that by means of the method in accordance with the invention the effectiveness of secondary treatment can be predicted post-surgery for patients having a low- or high-risk profile. Preferably, the secondary treatment comprises one or more, more preferably all, of chemotherapy, hormone therapy and radiation therapy.
The method as provided herein is based on the expression levels, e.g. expression profiles, of at least one gene selected from immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2. It is appreciated that the method may be performed on an input relating to the expression levels of the one or more genes, or that determining the expression levels may be part of the method.

It is further envisioned that the method is performed by a processor. Therefore, in an embodiment the invention relates to a computer implemented method of predicting an outcome of a bladder cancer subject.

It is preferred that the method for predicting the outcome of a bladder cancer subject comprises determining the gene expression profile(s) of one or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of one or more, for example, 1, 2, 3, 4, 5, 6, 7, 8, 9, 10, 11, 12, 13, 14, 15, 16 or all, T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2.

In a further embodiment the method for predicting the outcome of a bladder cancer subject comprises determining the gene expression profile(s) of two or more, for example, 1, 2, 3, 4, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or of two or more, for example, 1, 2, 3, 4, 5, 6, 7, 8, 9, 10, 11, 12, 13, 14, 15, 16 or all, T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or of two or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2.

In one preferred embodiment the method according to the invention comprises that

- the one or more immune defense response genes comprise three or more, preferably, six or more, more preferably, nine or more, most preferably, all of the immune defense genes, and/or
- the one or more T-Cell receptor signalling genes comprise three or more, preferably, six or more, more preferably, nine or more, most preferably, all of the T-Cell receptor signalling genes, and/or
- the one or more PDE4D7 correlated genes comprise three or more, preferably, six or more, most preferably, all of the PDE4D7 correlated genes.

Thus, it is preferred that the determining the first, second and/or third gene expression profile(s) according to the method of the invention comprises the determining or receiving the result of a determination of

- three or more, preferably six or more, more preferably, nine or more, most preferably, all of the genes selected from immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1,
- three or more, preferably six or more, more preferably, nine or more, most preferably, all of the genes selected from the group and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
- three or more, preferably six or more, more preferably, nine or more, most preferably, all of the genes PDE4D7 correlated genes selected from the group consisting of: ABCC5, CUX2, KIAA1549, PDE4D, RAP1GAP2, SLC39A11, TDRD1, and VWA2.

In a further embodiment the method according to the invention comprises that the determining the prediction of the outcome is based on one or more immune defense response gene, one or more T-Cell receptor signalling gene and one or more PDE4D7 correlated gene. In a further embodiment the method according to the invention comprises that the determining the prediction of the outcome is based on two or more immune defense response genes, two or more T-Cell receptor signalling genes and two or more PDE4D7 correlated genes. In a further embodiment the method according to the invention comprises that the determining the prediction of the outcome is based on three or more immune defense response genes, three or more T-Cell receptor signalling genes and three or more PDE4D7 correlated genes.

As a first reference example, a (first) gene expression profile as embodied herein can comprise at least one, at least two, at least three immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1. In one non-limiting example, the gene expression profile as embodied herein comprises the expression profiles of the genes DDX58, DHX9 and IFI16.

As a further reference example, a (second) gene expression profile as embodied herein can comprise at least one, at least two, at least three T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70. In one non-limiting example, the gene expression profile as embodied herein comprises the expression profiles of the genes PRKACA, PRKACB and PTPRC.

As a further reference example, a (third) gene expression profile as embodied herein can comprise at least one, at least two, at least three PDE4D7 correlated genes selected from the group consisting of: ABCC5, CUX2, KIAA1549, PDE4D, RAP1GAP2, SLC39A11, TDRD1, and VWA2. In one non-limiting example, the gene expression profile as embodied herein comprises the expression profiles of the genes KIAA1549, PDE4D and RAP1GAP2.

As a further non-limiting reference example, a gene expression profile as provided herein may comprise a first, second and third gene expression profile, wherein the first gene expression profile comprises at least one, at least two, at least three immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, the second gene expression profile comprises at least one, at least two, at least three T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70 and the third gene expression profile comprises at least one, at least two, at least three PDE4D7 correlated genes selected from the group consisting of: ABCC5, CUX2, KIAA1549, PDE4D, RAP1GAP2, SLC39A11, TDRD1, and VWA2.

In a further non-limiting reference example, a gene expression profile for the prediction of an outcome of a bladder cancer can consist of the gene expression profiles of TLR8 (an IDR gene), CD2 (a TCR gene) and PDE4D (a PDE4D7 correlated gene). In another example, the gene expression profile can consist of the gene expression profiles of OAS1 and TLR8 (IDR genes), CD2 (a TCR gene) and PDE4D (a PDE4D7 correlated gene), or of the gene expression profiles of TLR8 (an IDR gene), CD2 and PTPRC (TCR genes) and PDE4D (a PDE4D7 correlated gene), or of the gene expression profiles of TLR8 (an IDR gene), CD2 (a TCR genes) and CUX2 and PDE4D (PDE4D7 correlated genes).

Cox proportional-hazards regression allows analysing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g. death or disease recurrence) is called the hazard. Next to the information on whether or not the tested endpoint was reached by e.g. subject in a patient cohort (e.g. patient did die or not) also the time to the endpoint is considered in the regression analysis. The hazard is modelled as: $H(t) = Ho(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (HR) (or the risk to reach the event) is represented by $Ln[H(t)/ Ho(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

Therefore in one preferred embodiment the method according to the invention comprises that the determining of the prediction of the outcome comprises combining the combination of the first gene expression profile, the combination of the second gene expression profiles and the combination of the third gene expression profiles with a regression function that has been derived from a population of bladder cancer subjects.

In another preferred embodiment the method according to the invention comprises that the determining of the prediction of the outcome comprises:

- combining the first gene expression profiles for two or more, for example, 2, 3, 4, 5, 6, 7, 8, 9, 10, 11, 12, 13 or all, of the immune defense response genes with a regression function that has been derived from a population of bladder cancer subjects, and/or
- combining the second gene expression profiles for two or more, for example, 2, 3, 4, 5, 6, 7, 8, 9, 10, 11, 12, 13, 14, 15, 16 or all, of the T-Cell receptor signalling genes with a regression function that has been derived from a population of bladder cancer subjects, and/or
- combining the third gene expression profiles for two or more, for example, 2, 3, 4, 5, 6, 7 or all, of the PDE4D7 correlated genes with a regression function that has been derived from a population of bladder cancer subjects.

In one particular realization, the prediction of the outcome is determined as follows:

$$IDR\_14\_model: \hspace{4cm} (1)$$

$$(w_1 \cdot AIM2) + (w_2 \cdot APOBEC3A) + (w_3 \cdot CIAO1) + [...] + (w_{14} \cdot ZBP1)$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of genes.

In another particular realization, the prediction of the outcome is determined as follows:

TCR_17_model: (2)

$$(w_{15} \cdot CD2) + (w_{16} \cdot CD247) + (w_{17} \cdot CD28) + [\ldots] + (w_{31} \cdot ZAP70)$$

where $w_{15}$ to $w_{31}$ are weights and CD2, CD247, CD28, CD3E, CD3G, CD4, CSK, EZR, FYN, LAT, LCK, PAG1, PDE4D, PRKACA, PRKACB, PTPRC, and ZAP70 are the expression levels of genes.

In another particular realization, the prediction of the outcome is determined as follows:

PDE4D7_CORR_model: (3)

$$(w_{32} \cdot ABCC5) + (w_{33} \cdot CUX2) + (w_{34} \cdot KIAA1549) + [\ldots] + (w_{39} \cdot VWA2)$$

where $w_{32}$ to $w_{39}$ are weights and ABCC5, CUX2, KIAA1549, PDE4D, RAP1GAP2, SLC39A11, TDRD1, and VWA2 are the expression levels of genes.

In another particular realization, the prediction of the outcome is determined as follows:

BCAI_model (4)

$$(w_{42} \cdot PDE4D7\_CORR) + (w_{40} \cdot IDR\_14) + (w_{41} \cdot TCR\_17)$$

In another realization, the prediction of the outcome is determined as follows:

BCAI_model (5)

$$(w_1 \cdot AIM2) + (w_2 \cdot APOBEC3A) + (w_3 \cdot CIAO1) + [\ldots] + (w_{14} \cdot ZBP1) + (w_{15} \cdot CD2) +$$
$$(w_{16} \cdot CD247) + (w_{17} \cdot CD28) + [\ldots] + (w_{31} \cdot ZAP70) + (w_{32} \cdot ABCC5) + (w_{33} \cdot CUX2) + (w34 \cdot$$
$$KIAA1549) + [\ldots] + (w_{39} \cdot VWA2),$$

wherein $w_1$ to $w_{39}$ are weights and AIM2, APOBEC3A, CIAO1, DDX58, DHX9, IFI16, IFIH1, IFIT1, IFIT3, LRRFIP1, MYD88, OAS1, TLR8, ZBP1, CD2, CD247, CD28, CD3E, CD3G, CD4, CSK, EZR, FYN, LAT, LCK, PAG1, PDE4D, PRKACA, PRKACB, PTPRC, ZAP70, ABCC5, CUX2, KIAA1549, PDE4D, RAP1GAP2, SLC39A11, TDRD1, and VWA2 are the expression levels of genes.

The prediction of outcome may also be classified or categorized into one of at least two risk groups, based on the value of the prediction of the outcome. For example, there may be two risk groups, or three risk groups, or four risk groups, or more than four predefined risk groups.

Each risk group covers a respective range of (non-overlapping) values of the prediction of the outcome. For example, a risk group may indicate a probability of occurrence of a specific clinical event from 0 to <0.1 or from 0.1 to <0.25 or from 0.25 to <0.5 or from 0.5 to 1.0 or the like.

In a preferred embodiment the method according to the invention comprises that the determining of the prediction of the outcome is based on one or more clinical parameters obtained from the subject. As mentioned above, various measures based on one or more clinical parameters have been investigated. By predicating the prediction of the outcome upon such clinical parameter(s), it can be possible to further improve the prediction.

In a preferred embodiment the one or more clinical parameters at least comprises the number of tumour positive lymph nodes. More preferably the clinical parameter is the number of tumour positive lymph nodes

In an embodiment the determining of the prediction of the therapy response comprises combining the gene expression levels for one or more IDR genes, one or more TCR signalling genes and/or one or more PDE4D7 correlated genes with a regression function that has been derived from a population of bladder cancer subjects.

It is further preferred that the gene expression profiles for the one or more IDR genes, one or more TCR signalling genes and/or one or more PDE4D7 correlated genes and the one or more clinical parameters obtained from the subject are combined with a regression function that had been derived from a population of bladder cancer subjects. Therefore in an embodiment the determining of the prediction of the outcome is further based on one or more clinical parameters obtained from the subject.

It is also preferred that the one or more clinical parameters are combined with one or more of the first, second and third gene expression(s) with a regression function that is derived from a bladder cancer subject population in other to provide for a method for predicting an outcome of a bladder cancer subject. Therefore in an embodiment the determining of the outcome comprises combining the gene expression profile and one or more clinical parameters obtained from the subject with a regression function that has been derived from a population of bladder cancer subjects.

As such, in a preferred embodiment of the method according to the invention that the determining of the outcome comprises combining one or more of:

(i) the first gene expression profile(s) for the one or more immune defense response genes;
(ii) the second gene expression profile(s) for the one or more T-Cell receptor signalling genes;
(iii) the third gene expression profile(s) for the one or more PDE4D7 correlated genes, and;
(iv) the combination of the first gene expression profiles, second gene expression profiles, the third gene expression profiles, and the one or more clinical parameters obtained from the subject with a regression function that has been derived from a population of bladder cancer subjects.

In another particular realization, the prediction of the outcome is determined as follows:

$$\text{BCAI\_Clinical Model} \qquad\qquad (6)$$

$$(w_{43} \cdot \text{BCAI\_model}) + (w_{44} \cdot \text{LN\_positive})$$

where $w_{43}$ and $w_{44}$ are weights, BCAI_model is the above described regression model based on the expression profiles of one or more IDR genes, one or more TCR signalling genes and/or one or more PDE4D7 correlated genes, and LN_positive represents the number of tumour positive lymph nodes. Multi-variate Cox regression was used to create the clinical model combining the BCAI with clinical data (number of lymph node positive).

An example of a suitable clinical parameter, as has been used and exemplified herein, is "LN_positive" representing the number of tumour positive lymph nodes after post-surgical pathology. A LN positive bladder tumour is a tumour of the bladder wherein tumour cells have spread, i.e. metastasized, to nearby lymph nodes. It is contemplated that LN positive tumours are a harbinger for subclinical metastasis of the cancer.

Additional examples of clinical parameters are the number of tumour positive regional lymph nodes, metastatic disease status, ECOG score, and FoundationOne mutation burden per MB DNA. Therefore in an embodiment the one or more clinical parameters comprise one or more of: the number of tumour positive regional lymph nodes, metastatic disease status, ECOG score, and FoundationOne mutation burden per MB DNA, preferably wherein the one or more clinical parameters consist of the number of tumour positive regional lymph nodes, or consist of metastatic disease status, ECOG score, and optionally FoundationOne mutation burden per MB DNA.

The number of tumour positive regional lymph nodes when used herein reflects a score where lymph nodes in the region of the bladder are examined to have tumors, where a score of 0 reflects no tumor positive regional lymph nodes and any integer above 0 reflects the number of tumor positive lymph nodes identified. Generally lymph nodes with only isolated tumor cells (ITCs) are not counted as positive lymph nodes, only lymph nodes with metastases greater than 0.2mm (micrometastases or larger) are counted as positive.

When used herein the term metastatic disease status refers to the staging of tumors in categories I-IV generally used by clinicians. Commonly the stages are classified as: Stage I: Cancer is localized to a small area and hasn't spread to lymph nodes or other tissues. Stage II: Cancer has grown, but it hasn't spread. Stage III: Cancer has grown larger and has possibly spread to lymph nodes or other tissues. Stage IV: Cancer has spread to other organs or areas of your body.

When used herein, the term ECOG score is understood to have its usual meaning. The ECOG score describes a patient's level of functioning in terms of their ability to care for themself, daily activity, and physical ability (walking, working, etc.). The scale was developed by the Eastern Cooperative Oncology Group (ECOG) and published in 1982; it is also called the WHO or Zubrod score and runs from 0 to 5, with 0 denoting perfect health and 5 death. The score is assigned as:

0 - Asymptomatic. Fully active, able to carry on all predisease activities without restriction.
1 - Symptomatic but completely ambulatory. Restricted in physically strenuous activity but ambulatory and able to carry out work of a light or sedentary nature (e.g., light housework, office work).
2 - Symptomatic, <50% in bed during the day. Ambulatory and capable of all self-care but unable to carry out any work activities. Up and about more than 50% of waking hours.
3 - Symptomatic, >50% in bed, but not bedbound. Capable of only limited self-care, confined to bed or chair 50% or more of waking hours.

4 - Bed bound. Completely disabled. Cannot carry on any self-care. Totally confined to bed or chair.

5 - Death.

When used herein the term FoundationOne mutation burden per MB DNA is defined as the number of somatic mutations per megabase of interrogated genomic sequence and as determined using the FoundationOne CDx Next generation sequencing panel.

It is understood that for NMIBC or MIBC, the used clinical parameter preferably includes or is number of tumour positive regional lymph nodes. In metastatic bladder cancer the clinical parameters include or are preferably metastatic disease status, ECOG score, optionally complemented with FoundationOne mutation burden per MB DNA.

The method according to the invention may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

In a preferred embodiment the invention therefore also provides for a computer program product comprising instructions which, when the program is executed by a computer, cause the computer to carry out a method comprising:

- receiving data indicative of a gene expression profile comprising three or more, for example, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or even 39, gene expression levels, wherein the three or more gene expression levels are selected from: immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or PDE4D7 correlated genes selected from the group consisting of: ABCC5, CUX2, KIAA1549, PDE4D, RAP1GAP2, SLC39A11, TDRD1, and VWA2, said gene expression profile being determined in a biological sample obtained from the subject determining the prediction of the outcome based on the gene expression profile, wherein said prediction is a favourable outcome or a non-favourable outcome for the subject. Optionally, the program product further comprising instructions for providing the prediction of the outcome to a medical caregiver or the subject.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the steps described herein can be used to implement one or more steps of the method of risk stratification for therapy selection in a patient with bladder cancer is illustrated. As will be appreciated, while the steps of the method may all be computer implemented, in some embodiments one or more of the steps may be at least partially performed manually.

It is preferred herein that the computer program product according to the invention can be implemented on an apparatus for predicting an outcome of a bladder cancer subject, wherein the apparatus comprises an input adapted to receive data indicative of gene expression profile(s) of immune defense response genes and/or T-Cell receptor signalling genes and/or PDE4D7 correlated genes, and wherein said apparatus further comprises a processor adapted to determine the prediction of an outcome based on said one or more gene expression profile(s), and

- optionally, a providing unit adapted to provide the prediction or provide a therapy recommendation based on the selection to a medical caregiver or to the subject.

In a further aspect of the present invention is provided for an apparatus for predicting an outcome of a bladder cancer subject, comprising:

- an input adapted to receive data indicative of a gene expression profile comprising three or more, for example, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or even 39, gene expression levels, wherein the three or more gene expression levels are selected from:

- immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
- T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
- PDE4D7 correlated genes selected from the group consisting of: ABCC5, CUX2, KIAA1549, PDE4D, RAP1GAP2, SLC39A11, TDRD1, and VWA2,

said gene expression profile being determined in a biological sample obtained from the subject,

- a processor adapted to determine the prediction of the radiotherapy response based on the gene expression profile(s) for the three or more genes, and
- optionally, a providing unit adapted to provide the prediction or a therapy recommendation based on the prediction to a medical caregiver or the subject.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

In a preferred embodiment the invention therefore also provides for a diagnostic kit, comprising at least one polymerase chain reaction primer, and optionally at least one probes, for determining the gene expression profile(s) in a biological sample obtained from a bladder cancer subject and/or in a sample.

It is preferred that said diagnostic kit as provided herein comprises at least one of: a polymerase chain reaction primer or probes, for determining a gene expression profile, the gene expression profile comprising three or more expression levels in a biological sample obtained from a bladder cancer subject and/or in a sample, wherein the three or more gene expression levels are selected from:

- immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
- T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
- PDE4D7 correlated genes selected from the group consisting of: ABCC5, CUX2, KIAA1549, PDE4D, RAP1GAP2, SLC39A11, TDRD1, and VWA2. The kit may be a PCR kit, a quantitative PCR kit, an RNA sequencing kit, a targeted RNA sequencing kit, a Serial Analysis of Gene Expression (SAGE) kit, a DNA microarray, or a Tiling array.

In another preferred embodiment the invention provides for the use of a diagnostic kit as broadly embodied herein in a method of predicting an outcome of a bladder cancer subject, preferably for use in the method for predicting the outcome of a bladder cancer subject as broadly embodied herein. Therefore, in an embodiment the invention relates to use of a diagnostic kit, the kit comprising:

- at least one of: a polymerase chain reaction primer or probes, for determining a gene expression profile, the gene expression profile comprising three or more expression levels in a biological sample obtained from a bladder cancer subject and/or in a sample, wherein the three or more gene expression levels are selected from:

  - immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
  - T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
  - PDE4D7 correlated genes selected from the group consisting of: ABCC5, CUX2, KIAA1549, PDE4D, RAP1GAP2, SLC39A11, TDRD1, and VWA2,

the use comprising predicting an outcome of a bladder cancer subject. Preferably the use comprises using the kit in the method for predicting an outcome as broadly defined herein.

In another preferred embodiment the invention provides for a method, comprising:

- receiving a biological sample obtained from a bladder cancer subject,
- using the diagnostic kit, as broadly embodied herein, to determining a gene expression profile, the gene expression profile comprising three or more expression levels in a biological sample obtained from a bladder cancer subject and/or in a sample, wherein the three or more gene expression levels are selected from:

- immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
- T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
- PDE4D7 correlated genes selected from the group consisting of: ABCC5, CUX2, KIAA1549, PDE4D, RAP1GAP2, SLC39A11, TDRD1, and VWA2, in a biological sample obtained from a bladder cancer subject and/or in a sample.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

Other variations to the disclosed realizations can be understood and effected by those skilled in the art in practicing the claimed invention, from a study of the drawings, the disclosure, and the appended claims.

Any reference signs in the claims should not be construed as limiting the scope.

EXAMPLES

*Example 1: Predicting the survival outcome for non-metastatic (M0) bladder cancer subjects*

Multiple data sets were used for the analysis of the three gene signatures. The TCGA (The Genome Cancer Atlas) created gene expression data together with clinical and survival data was downloaded from the database TCGA Bladder Urothelial sCarcinoma, Firehose legacy, accessed March 6, 2020. This cohort comprises 412 patients with a combination of primary, localized (mostly consisting of muscle-invasive bladder cancer) and metastatic disease (i.e., patients with M>0 disease). The median follow-up in this cohort was 18 months (max 13 years) after the initial cancer diagnosis.

The TCGA gene expression values were presented as log2 data. The log2_expression values for each gene were transformed into z-scores by calculating:

$$\text{z-score log2\_gene} = ((\text{log2\_gene})-(\text{mean\_samples}))/(\text{stdev\_samples}) \qquad (7)$$

Where log2_gene is the log2 gene expression value per gene; mean_samples is the mathematical mean of log2_gene values across all samples and stdev _samples is the standard deviation of the log2_gene values across all samples.

This process distributes the transformed log2_gene values around the mean 0 with a standard deviation of 1. For the multivariate analysis of the genes of interest the log2_gene transformed z-score value of each gene as input as used.

Cox Regression Analysis

We selected 196 patients of the entire TCGA data set with M0 disease of which 189 had survival data available. We split this group into 95 patients for training of the three gene signatures and to build the bladder cancer ImmunoScore (BCAI) to prognosticate the overall survival of patients after the initial diagnosis of the disease.

Provided herein is a test to assess whether the combination of the 14 immune defense response genes, the combination of the 17 T-Cell receptor signalling genes, the combination of the 8 PDE4D7 correlated genes, and a combination thereof will exhibit a prognostic value for bladder cancer. By using Cox regression, the expression levels of the 14 immune defense response genes, of the 17 T-Cell receptor signalling genes, and of the eight PDE4D7 correlated genes, respectively, were modelled to overall survival.

The Cox regression functions for the individual gene signatures were derived as follows:

IDR_14_model: (8)

$$(w_1 \cdot AIM2) + (w_2 \cdot APOBEC3A) + (w_3 \cdot CIAO1) + (w_4 \cdot DDX58) +$$
$$(w_5 \cdot DHX9) + (w_6 \cdot IFI16) + (w_7 \cdot IFIH1) + (w_8 \cdot IFIT1) + (w_9 \cdot IFIT3)$$
$$+ (w_{10} \cdot LRRFIP1) + (w_{11} \cdot MYD88) + (w_{12} \cdot OAS1) + (w_{13} \cdot TLR8) +$$
$$(w_{14} \cdot ZBP1)$$

TCR_17_model: (9)

$$(w_{15} \cdot C2) + (w_{16} \cdot CD247) + (w_{17} \cdot CD28) + (w_{18} \cdot CD3E) + (w_{19} \cdot$$
$$CD3G) + (w_{20} \cdot CD4) + (w_{21} \cdot CSK) + (w_{22} \cdot EZR) + (w_{23} \cdot FYN) + (w_{24}$$
$$\cdot LAT) + (w_{25} \cdot LCK) + (w_{26} \cdot PAG1) + (w_{27} \cdot PDE4D) + (w_{28} \cdot$$
$$PRKACA) + (w_{29} \cdot PRKACB) + (w_{30} \cdot PTPRC) + (w_{31} \cdot ZAP70)$$

PDE4D7_CORR_model: (10)

$$(w_{32} \cdot ABCC5) + (w_{33} \cdot CUX2) + (w_{34} \cdot KIAA1549) + (w_{35} \cdot PDE4D) +$$
$$(w_{36} \cdot RAP1GAP2) + (w_{37} \cdot SLC39A11) + (w_{38} \cdot TDRD1) + (w_{39} \cdot$$
$$VWA2)$$

The details for the weights $w_1$ to $w_{39}$ are shown in the following Table 4.

Table 4: Variables and weights for the three individual Cox regression models, i.e. the immune defense response model (IDR model), the T-Cell receptor signalling model (TCR SIGNALING model), and the PDE4D7 correlation model (PDE4D7_CORR_model) for bladder cancer; N/A - not available.

| Variable | | Weights | | |
|---|---|---|---|---|
| Model | | IDR model | TCR_ SIGNALING_ model | PDE4D7_ CORR_ model |
| AIM2 | $w_1$ | -0,5942 | N/A | N/A |
| APOBEC3A | $w_2$ | 0,01637 | N/A | N/A |
| CIAO1 | $w_3$ | -0,2327 | N/A | N/A |
| DDX58 | $w_4$ | -0,6255 | N/A | N/A |
| DHX9 | $w_5$ | 0,5305 | N/A | N/A |
| IFI16 | $w_6$ | 0,2643 | N/A | N/A |
| IFIH1 | $w_7$ | -0,06682 | N/A | N/A |
| IFIT1 | $w_8$ | 0,4733 | N/A | N/A |
| IFIT3 | $w_9$ | 0,2698 | N/A | N/A |
| LRRFIP1 | $w_{10}$ | 0,3025 | N/A | N/A |
| MYD88 | $w_{11}$ | -0,2302 | N/A | N/A |

(continued)

| Variable | | Weights | | |
|---|---|---|---|---|
| Model | | IDR model | TCR_ SIGNALING_ model | PDE4D7_ CORR_ model |
| OAS1 | $w_{12}$ | -0,4159 | N/A | N/A |
| TLR8 | $w_{13}$ | 0,08662 | N/A | N/A |
| ZBP1 | $w_{14}$ | 0,07214 | N/A | N/A |
| CD2 | $w_{15}$ | N/A | 0,3242 | N/A |
| CD247 | $w_{16}$ | N/A | 0,03061 | N/A |
| CD28 | $w_{17}$ | N/A | 0,4636 | N/A |
| CD3E | $W_{18}$ | N/A | -0,3372 | N/A |
| CD3G | $w_{19}$ | N/A | -0,09887 | N/A |
| CD4 | $w_{20}$ | N/A | 0,1825 | N/A |
| CSK | $w_{21}$ | N/A | -0,1961 | N/A |
| EZR | $w_{22}$ | N/A | -0,03954 | N/A |
| FYN | $w_{23}$ | N/A | 0,1259 | N/A |
| LAT | $w_{24}$ | N/A | -0,06429 | N/A |
| LCK | $w_{25}$ | N/A | -0,07393 | N/A |
| PAG1 | $w_{26}$ | N/A | -0,705 | N/A |
| PDE4D | $w_{27}$ | N/A | 0,3454 | N/A |
| PRKACA | $w_{28}$ | N/A | 0,4765 | N/A |
| PRKACB | $w_{29}$ | N/A | 0,2676 | N/A |
| PTPRC | $w_{30}$ | N/A | 0,6749 | N/A |
| ZAP70 | $w_{31}$ | N/A | -0,8557 | N/A |
| ABCC5 | $w_{32}$ | N/A | N/A | -0,112 |
| CUX2 | $w_{33}$ | N/A | N/A | -0,3117 |
| KIAA1549 | $w_{34}$ | N/A | N/A | -0,00355 |
| PDE4D | $w_{35}$ | N/A | N/A | 0,2887 |
| RAP1GAP2 | $w_{36}$ | N/A | N/A | -0,08836 |
| SLC39A11 | $w_{37}$ | N/A | N/A | -0,1632 |
| TDRD1 | $w_{38}$ | N/A | N/A | 0,2415 |
| VWA2 | $w_{39}$ | N/A | N/A | 0,209 |

Finally, the three individual gene signatures were combined by Cox regression analysis using overall survival as the clinical endpoint to predict. The Cox regression functions were derived as follows:

BCAI_model (Bladder Cancer ImmunoScore): $\qquad$ (11)

$$(w_{40} \cdot IDR\_14\_model) + (w_{41} \cdot TCR\_17\_model) + (w_{42} \cdot PDE4D7\_CORR\_model)$$

The BCAI_Clinical Score was built on the TCGA discovery cohort (95 patients; see above) and tested on the TCGA validation cohort (94 patients; see above). The clinical endpoint tested in MV Cox regression was overall death. As input for the MV Cox regression analysis the IDR, TCR, and PDE4D7_CORR signature scores as derived from the individual Cox regression models were used, as well as the number of tumour positive lymph nodes (LN_positive) as determined in post-surgical pathology. This results in the final BCAI _Clinical Score.

BCAI_Clinical_model (bladder cancer): $\qquad$ (12)

$$(w_{43} \cdot IDR\_14\_model) + (w_{44} \cdot TCR\_17\_model) + (w_{45} \cdot PDE4D7\_CORR\_model) + (w_{46} \cdot LN\_stage=1) + (w_{47} \cdot LN\_stage=2) + (w_{48} \cdot LN\_stage=3)$$

The details for the weights $w_{40}$ to $w_{44}$ are shown in the following table 5. BCAI_Clinical_model is herein also referred to as BCAI&Clinical_model.

Table 5: Variables and weights for two combination Cox regression models, i.e. bladder cancer AI model (BCAI_model) and the bladder & clinical model (BCAI&Clinical model); N/A - not available.

| Variable | Weights | | |
|---|---|---|---|
| **Model** | | **BCAI_model** | **BCAI&Clinical_model** |
| IDR_14_model | $w_{40}$ | 0,7153 | N/A |
| TCR_17_model | $w_{41}$ | 0,6342 | N/A |
| PDE4D7_CORR_model | $w_{42}$ | 0,2713 | N/A |
| IDR_14_model | $w_{43}$ | N/A | 0,812 |
| TCR_17_model | $w_{44}$ | N/A | 0,6421 |
| PDE4D7_CORR_model | $w_{45}$ | N/A | 0,3035 |
| pathology_N_stage="N1" | $w_{46}$ | N/A | 0,8696 |
| pathology_N_stage="N2" | $w_{47}$ | N/A | 0,1511 |
| pathology_N_stage="N3" | $w_{48}$ | N/A | 2,4923 |

For further validation of the BCAI and BCAI_Clinical models we combined the data sets GSE13507, GSE32894, UROMOL after z-score transformation of the log2 expression values of the genes representing the three gene signatures with the 94 samples of TCGA data which in the first step as described above were used for validation of the BCAI and BCAI_Clinical models. This super data set consists of 1,262 bladder cancer patients in total.

**Kaplan-Meier Survival Analysis**

The two models (BCAI, and BCAI&Clinical) as developed on the TCGA discovery cohort were tested with Kaplan Meier analysis on the TCGA validation cohort as well as on the combination super data set. Different clinical endpoints were tested (cancer specific death; overall death; death after treatment).

For Kaplan-Meier survival curve analysis, the Cox functions of the tested risk models (BCAI model, BAI&Clinical_model) were categorized into two sub-cohorts based on a cut-off. The threshold for group separation into low and high risk was based on the risk to experience the clinical endpoint (outcome) as predicted by the respective Cox regression model.

Figures 4 - 8 display the Kaplan-Meier survival curve analyses of the TCGA training and validation cohort for the PDE4D7_R2, IDR_14, TCR_17, BCAI and BCAI_Clinical models respectively.

Figures 9 - 18 display the Kaplan-Meier survival curve analyses of the super data set validation cohort.

The Kaplan-Meier analyses as shown in the one or more Figs. 4 - 8, and Figs. 9 - 18 demonstrate that an outcome, e.g., selected from bladder cancer specific death; overall death of a bladder cancer subject can be predicted. By using risk models, that are based on a randomly selected combinations of genes as provided herein, is provided for an improved prediction of the outcome of a bladder cancer subject. The prediction of the outcome improves therapy selection and potentially survival. By using the herein developed risk model, it can be expected to improve the prediction of the effectiveness of therapy options post-surgery in said subject. Further, this would reduce suffering for those patients who would be spared ineffective therapy and would reduce cost spent on ineffective therapies. Overall, based on the models comprising the variables and weights of genes as presented herein, an outcome of a bladder cancer subject can be methodologically, for example through distinguishing the risk of a particular outcome in different patient risk groups by means of stratification and statistical methodology, predicted.

*Example 2: Predicting the survival outcome in metastatic bladder cancer after immunotherapy with an anti-PD-L1 immune checkpoint inhibitor*

For the analysis of survival outcome prediction in metastatic bladder cancer after immune checkpoint inhibitor treatment the processed data of the clinical phase 2 trial was used (https://doi.org/10.1016/s0140-6736(16)32455-2). The total patient cohort consisted of 348 patients of which 298 were eligible for survival analysis due to complete data on treatment response. This cohort was split into a training (224 patients) and a test set (74 patients). The same procedure for the training of the three gene signatures and their combination as outlined above were used to train a metastatic BCAI (metBCAI) signature. Additionally available clinical features on metastatic disease stage (classified as lymph nodes, liver, or visceral metastases) as well as baseline ECOG (Eastern Cooperative Oncology Group) performance scores were used to build the metBCAI_Clinical ImmunoScore.

Cox regression models

Provided herein is a test to assess whether the combination of the 14 immune defense response genes, the combination of the 17 T-Cell receptor signalling genes, the combination of the 8 PDE4D7 correlated genes, and a combination thereof will exhibit a prognostic value for metastatic bladder cancer after the treatment with the anti-PD-L1 immune checkpoint inhibitor atezoluzimab. By using Cox regression, the expression levels of the 14 immune defense response genes, of the 17 T-Cell receptor signalling genes, and of the eight PDE4D7 correlated genes, respectively, were modelled to overall survival.

The Cox regression functions for the individual gene signatures were derived as follows:

metIDR_14_model: (13)

$$(w_{49} \cdot AIM2) + (w_{50} \cdot APOBEC3A) + (w_{51} \cdot CIAO1) + (w_{52} \cdot DDX58) +$$
$$(w_{53} \cdot DHX9) + (w_{54} \cdot IFI16) + (w_{55} \cdot IFIH1) + (w_{56} \cdot IFIT1) + (w_{57} \cdot IFIT3) + (w_{58} \cdot LRRFIP1) + (w_{59} \cdot MYD88) + (w_{60} \cdot OAS1) + (w_{61} \cdot TLR8) + (w_{62} \cdot ZBP1)$$

metTCR_17_model: (14)

$$(w_{63} \cdot C2) + (w_{64} \cdot CD247) + (w_{65} \cdot CD28) + (w_{66} \cdot CD3E) + (w_{67} \cdot CD3G) + (w_{68} \cdot CD4) + (w_{69} \cdot CSK) + (w_{70} \cdot EZR) + (w_{71} \cdot FYN) + (w_{72} \cdot LAT) + (w_{73} \cdot LCK) + (w_{74} \cdot PAG1) + (w_{75} \cdot PDE4D) + (w_{76} \cdot PRKACA) + (w_{77} \cdot PRKACB) + (w_{78} \cdot PTPRC) + (w_{79} \cdot ZAP70)$$

metPDE4D7_CORR_model: (15)

$$(w_{80} \cdot ABCC5) + (w_{81} \cdot CUX2) + (w_{82} \cdot KIAA1549) + (w_{83} \cdot PDE4D) +$$
$$(w_{84} \cdot RAP1GAP2) + (w_{85} \cdot SLC39A11) + (w_{86} \cdot TDRD1) + (w_{87} \cdot VWA2)$$

The details for the weights $w_1$ to $w_{39}$ are shown in the following Table 6.

Table 6: Variables and weights for the three individual Cox regression models, i.e., the immune defense response model (metIDR model), the T-Cell receptor signalling model (metTCR SIGNALING model), and the PDE4D7 correlation model (metPDE4D7_CORR_model) for bladder cancer; N/A - not available.

| Variable Model | | Weights | | |
|---|---|---|---|---|
| | | metIDR_model | metTCR_ SIGNALING_ model | metPDE4D7_ CORR_ model |
| AIM2 | $W_{49}$ | 0,2171 | N/A | N/A |
| APOBEC3A | $W_{50}$ | 0,09108 | N/A | N/A |
| CIAO1 | $W_{51}$ | -0,1249 | N/A | N/A |
| DDX58 | $W_{52}$ | 0,04276 | N/A | N/A |
| DHX9 | $W_{53}$ | -0,1428 | N/A | N/A |
| IFI16 | $W_{54}$ | -0,1554 | N/A | N/A |
| IFIH1 | $W_{55}$ | 0,00153 | N/A | N/A |
| IFIT1 | $W_{56}$ | 0,6924 | N/A | N/A |
| IFIT3 | $W_{57}$ | -0,5596 | N/A | N/A |
| LRRFIP1 | $W_{58}$ | -0,07881 | N/A | N/A |
| MYD88 | $W_{59}$ | -0,07074 | N/A | N/A |
| OAS1 | $W_{60}$ | -0,07859 | N/A | N/A |
| TLR8 | $W_{61}$ | 0,02086 | N/A | N/A |
| ZBP1 | $W_{62}$ | -0,2024 | N/A | N/A |
| CD2 | $W_{63}$ | N/A | -0,3943 | N/A |
| CD247 | $W_{64}$ | N/A | 0,01382 | N/A |
| CD28 | $W_{65}$ | N/A | 0,3648 | N/A |
| CD3E | $W_{66}$ | N/A | -0,2889 | N/A |
| CD3G | $W_{67}$ | N/A | -0,05413 | N/A |
| CD4 | $W_{68}$ | N/A | 0,3501 | N/A |
| CSK | $W_{69}$ | N/A | 0,02689 | N/A |
| EZR | $W_{70}$ | N/A | -0,03889 | N/A |
| FYN | $W_{71}$ | N/A | -0,2402 | N/A |
| LAT | $W_{72}$ | N/A | 0,03648 | N/A |
| LCK | $W_{73}$ | N/A | -0,4952 | N/A |

(continued)

| Variable Model | | Weights | | |
|---|---|---|---|---|
| | | metIDR_model | metTCR_ SIGNALING_ model | metPDE4D7_ CORR_ model |
| PAG1 | $W_{74}$ | N/A | -0,04833 | N/A |
| PDE4D | $W_{75}$ | N/A | 0,05807 | N/A |
| PRKACA | $W_{76}$ | N/A | -0,1413 | N/A |
| PRKACB | $W_{77}$ | N/A | 0.1097 | N/A |
| PTPRC | $W_{78}$ | N/A | 0,08487 | N/A |
| ZAP70 | $W_{79}$ | N/A | 0,534 | N/A |
| ABCC5 | $W_{80}$ | N/A | N/A | 0,1381 |
| CUX2 | $W_{81}$ | N/A | N/A | 0,09451 |
| KIAA1549 | $W_{82}$ | N/A | N/A | -0,02703 |
| PDE4D | $W_{83}$ | N/A | N/A | 0,07988 |
| RAP1GAP2 | $W_{84}$ | N/A | N/A | -0,2537 |
| SLC39A11 | $W_{85}$ | N/A | N/A | -0,1437 |
| TDRD1 | $W_{86}$ | N/A | N/A | 0,0235 |
| VWA2 | $W_{87}$ | N/A | N/A | 0,001631 |

Finally, the three individual gene signatures were combined by Cox regression analysis using overall survival as the clinical endpoint to predict. The Cox regression functions were derived as follows:

BCAI_model (metastatic Bladder Cancer ImmunoScore):

$$(w_{88} \cdot \text{IDR\_14\_model}) + (w_{89} \cdot \text{TCR\_17\_model}) + (w_{90} \cdot \text{PDE4D7\_CORR\_model}) \qquad (16)$$

The BCAI_Clinical Score was built on the training cohort (224 patients; see above) and tested on the validation cohort (74 patients; see above). The clinical endpoint tested in MV Cox regression was overall death after treatment with atezoluzimab. As input for the MV Cox regression analysis the metIDR, metTCR, and metPDE4D7_CORR signature scores as derived from the individual Cox regression models were used, as well as the metastatic disease stage (lymph node (LN), liver, visceral metastases) and the ECOG performance score at baseline. This results in the final BCAI_Clinical Score.

$$\text{BCAI\_Clinical\_model (metastatic bladder cancer):} \qquad (17)$$
$$(w_{91} \cdot \text{BCAI\_model}) + (w_{92} \cdot \text{LN metastatic disease stage}) + (w_{93} \cdot \text{visceral metastatic disease stage}) + (w_{94} \cdot \text{ECOG performance score at baseline}).$$

The details for the weights $w_{88}$ to $w_{92}$ are shown in the following table 7. BCAI_Clinical_model is herein also referred to as BCAI&Clinical_model.

Table 7: Variables and weights for two combination Cox regression models, i.e. metastatic bladder cancer AI model (BCAI model) and the bladder & clinical model (BCAI&Clinical model); N/A - not available.

| Variable | Weights | | |
|---|---|---|---|
| Model | | metBCAI_model | metBCAI&Clinical_model |
| IDR_14_model | $W_{88}$ | 0,825 | N/A |
| TCR_17_model | $W_{89}$ | 0,7973 | N/A |
| PDE47_CORR_model | $W_{90}$ | 0,28 | N/A |
| metBCAI_model | $W_{91}$ | N/A | 0,8898 |
| Met_Disease_Status="2_Liver" | $W_{92}$ | N/A | 0,9077 |
| Met_Disease_Status="3_Visceral" | $W_{93}$ | N/A | 0,5671 |
| Baseline_ECOG_Score | $W_{94}$ | N/A | 0,4595 |

As another variation of the clinical BCAI model the mutation burden per mega base of DNA as measured by the commercial 'FMOne mutational burden test' (Foundation Medicine) was added into the regression model:

$$\text{BCAI\_Clinical\_MB\_model (metastatic bladder cancer):} \tag{18}$$

$$(w_{95} \cdot \text{BCAI\_model}) + (w_{96} \cdot \text{LN metastatic disease stage}) + (w_{97} \cdot$$

$$\text{visceral metastatic disease stage}) + (w_{98} \cdot \text{ECOG performance score at}$$

$$\text{baseline}) + (w_{99} \cdot \text{mutational burden per mega base DNA})$$

The details for the weights $w_{40}$ to $w_{47}$ are shown in the following table 9. BCAI_Clinical_model is herein also referred to as BCAI&Clinical_model.

Table 9: Variables and weights for combination Cox regression models, i.e. metastatic bladder cancer & clinical & MB AI model (BCAI&Clinical&MB_model); N/A - not available.

| Variable | Weights | |
|---|---|---|
| Model | | BCAI&Clinical&MB_model |
| metBCAI_model | $W_{95}$ | 0,8213 |
| Met_Disease_Status="2_Liver" | $W_{96}$ | 0,586 |
| Met_Disease_Status="3_Visceral" | $W_{97}$ | 0,3585 |
| Baseline_ECOG_Score | $W_{98}$ | 0,623 |
| mutational burden per mega base DNA | $W_{99}$ | -0,03026 |

## Kaplan-Meier Survival and AUROC Analysis

For Kaplan-Meier survival curve analysis, the Cox functions of the tested risk models (metBCAI _model, met-BCAI&Clinical model, metBCAI&Clinical&MB_model) were categorized into two sub-cohorts based on a cut-off. The threshold for group separation into low and high risk was based on the risk to experience the clinical endpoint (outcome) as predicted by the respective Cox regression model.

Figures 19 - 25 display the Kaplan-Meier survival curve analyses of the training and validation cohort.

Figures 26 - 27 display the AUROC (area under the receiver-operator curve) analyses of the training and validation cohort.

The Kaplan-Meier analyses as shown in the one or more Figs. 19 - 25 demonstrate that an outcome, e.g. selected from metastatic bladder cancer specific death; overall death of a metastatic bladder cancer subject treated with an immune checkpoint inhibitor can be predicted. By using risk models, that are based on a randomly selected combinations of genes as provided herein, is provided for an improved prediction of the outcome of a bladder cancer subject. The prediction of the outcome improves therapy selection and potentially survival. By using the herein developed risk model, it can be expected to improve the prediction of the effectiveness of therapy options post-surgery in said subject. Further, this would reduce suffering for those patients who would be spared ineffective therapy and would reduce cost spent on ineffective therapies. Overall, based on the models comprising the variables and weights of genes as presented herein, an outcome of a bladder cancer subject can be methodologically, for example through distinguishing the risk of a particular outcome in different patient risk groups by means of stratification and statistical methodology, predicted.

**The attached Sequence Listing, entitled 2022PF00548 SEQ LIST, is incorporated herein by reference, in its entirety.**

**Claims**

1. A method of predicting an outcome of a bladder cancer subject, comprising:

    - determining or receiving the result of a determination of a gene expression profile comprising three or more gene expression levels, wherein the three or more gene expression levels are selected from:

        - immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
        - T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
        - PDE4D7 correlated genes selected from the group consisting of: ABCC5, CUX2, KIAA1549, PDE4D, RAP1GAP2, SLC39A11, TDRD1, and VWA2,

    said gene expression profile being determined in a biological sample obtained from the subject,
    - determining the prediction of the outcome based on the gene expression profile,
    - wherein said prediction is a favourable outcome or a non-favourable outcome for the subject,
    - optionally, providing the prediction of the outcome to a medical caregiver or the subject.

2. The method according to claim 1, wherein the three or more genes comprise one or more immune defence response gene, one or more T-Cell receptor signalling gene and one or more PDE4D7 correlated gene.

3. The method according to claim 1 or 2, wherein said favourable outcome or non-favourable outcome is a probability of survival, overall survival, cancer free survival, overall death, or cancer specific death.

4. The method according to any one of the preceding claims, wherein said favourable outcome or non-favourable outcome is survival in response to a treatment.

5. The method according to claims 4, wherein the treatment is surgery or immunotherapy, preferably wherein the immunotherapy is Bacillus Calmette-Guerin (BCG) immunotherapy or immune checkpoint inhibitor therapy.

6. The method according to any one of the previous claims, wherein:

    - the one or more immune defense response genes comprise three or more, preferably, six or more, more preferably, nine or more, most preferably, all of the immune defense genes, and/or
    - the one or more T-Cell receptor signalling genes comprise three or more, preferably, six or more, more preferably, nine or more, most preferably, all of the T-Cell receptor signalling genes, and/or
    - the one or more PDE4D7 correlated genes comprise three or more, preferably, six or more, most preferably, all of the PDE4D7 correlated genes.

7. The method according to any one of the previous claims, wherein the determining of the prediction of the outcome comprises combining the three or more gene expression levels with a regression function that has been derived from a population of bladder cancer subjects.

8. The method according to any one of the preceding claims, wherein the determining of the prediction of the outcome is further based on one or more clinical parameters obtained from the subject.

9. The method according to any one of the preceding claims, wherein the determining of the outcome comprises combining the gene expression profile and one or more clinical parameters obtained from the subject with a regression function that has been derived from a population of bladder cancer subjects.

10. The method according to any one of the preceding claims, wherein the one or more clinical parameters comprise one or more of: the number of tumour positive regional lymph nodes, metastatic disease status, ECOG score, and FoundationOne mutation burden per MB DNA, preferably wherein the one or more clinical parameters consist of the number of tumour positive regional lymph nodes, or consist of metastatic disease status, ECOG score, and optionally FoundationOne mutation burden per MB DNA.

11. The method according to any one of the preceding claims, wherein the biological sample is obtained from the subject before the start of the therapy, preferably wherein the biological sample is a bladder sample or a bladder cancer sample.

12. The method according to any one of the preceding claims, wherein a therapy is recommended based on the prediction.

13. The method according to any one of the preceding claims, wherein the subject has muscle invasive bladder carcinoma (MIBC), Non-muscle invasive bladder carcinoma (NMIBC) or metastatic bladder carcinoma (mUC).

14. A computer program product comprising instructions which, when the program is executed by a computer, cause the computer to carry out a method comprising:

   - receiving data indicative of a gene expression profile comprising three or more gene expression levels, wherein the three or more gene expression levels are selected from:

      - immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
      - T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
      - PDE4D7 correlated genes selected from the group consisting of: ABCC5, CUX2, KIAA1549, PDE4D, RAP1GAP2, SLC39A11, TDRD1, and VWA2,
      said gene expression profile being determined in a biological sample obtained from a bladder cancer subject,

   - determining a prediction of an outcome of the subject based on the gene expression profile wherein said prediction is a favourable outcome or a non-favourable outcome.

15. Use of a diagnostic kit, the kit comprising:

   - at least one of: a polymerase chain reaction primer or probes, for determining a gene expression profile, the gene expression profile comprising three or more expression levels in a biological sample obtained from a bladder cancer subject and/or in a sample, wherein the three or more gene expression levels are selected from:

      - immune defence response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, and/or
      - T-Cell receptor signalling genes selected from the group consisting of: CD2, CD247, CD28, CD3E, CD3G, CD4, CSK, EZR, FYN, LAT, LCK, PAG1, PDE4D, PRKACA, PRKACB, PTPRC, and ZAP70, and/or
      - PDE4D7 correlated genes selected from the group consisting of: ABCC5, CUX2, KIAA1549, PDE4D, RAP1GAP2, SLC39A11, TDRD1, and VWA2,
      the use comprising predicting an outcome of a bladder cancer subject, preferably wherein the use comprises using the kit in the method as defined in any one of claims 1 to 13.

**Fig. 1**

**Fig. 2**

GSE13507 → 165 Patients

GSE32894 → 308 Patients

UROMOL → 476 Patients

TCGA → 408 Patients

**1,357 Bladder Cancer Patients**
- NMIBC (#781; 59.8%)
- MIBC (#525; 40.2%)
- BCG treatment (#181)
- Cystectomy (#440)
- Median survival: 24 months

**Testing[1] of the BCAI_TCGA & BCAI_Clinical_TCGA Models**
(cut-off: median of the expression scores)

*[1] The 95 TCGA samples used for model training were excluded in model testing*

**Fig. 3**

survival_status

Survival Time [months]
training set (#95 patients)
pathology_M_stage="m0"

Number at risk
Group: <=threshold (0,3)

| 71 | 38 | 19 | 8 | 4 | 4 | 2 | 1 | 1 | 0 |
|----|----|----|---|---|---|---|---|---|---|

Group: >threshold (0,3)

| 24 | 5 | 3 | 1 | 1 | 0 | 0 | 0 | 0 | 0 |
|----|---|---|---|---|---|---|---|---|---|

survival_status

Survival Time [months]
testing set (#94 patients)
pathology_M_stage="m0"

Number at risk
Group: <=threshold (0,3)

| 68 | 35 | 20 | 13 | 5 | 3 | 2 | 2 | 1 | 0 |
|----|----|----|----|---|---|---|---|---|---|

Group: >threshold (0,3)

| 26 | 10 | 3 | 2 | 1 | 1 | 1 | 1 | 1 | 0 |
|----|----|---|---|---|---|---|---|---|---|

## Fig. 4

Fig. 5

survival_status

Survival Time [months]
training set (#95 patients)
pathology_M_stage="m0"

Number at risk
Group: <=threshold (0)

| 38 | 19 | 8 | 4 | 2 | 2 | 1 | 1 | 1 | 0 |
|---|---|---|---|---|---|---|---|---|---|

Group: >threshold (0)

| 57 | 24 | 14 | 5 | 3 | 2 | 1 | 0 | 0 | 0 |
|---|---|---|---|---|---|---|---|---|---|

survival_status

Survival Time [months]
testing set (#94 patients)
pathology_M_stage="m0"

Number at risk
Group: <=threshold (0)

| 51 | 26 | 13 | 8 | 2 | 1 | 1 | 1 | 1 | 0 |
|---|---|---|---|---|---|---|---|---|---|

Group: >threshold (0)

| 43 | 19 | 10 | 7 | 4 | 3 | 2 | 2 | 1 | 0 |
|---|---|---|---|---|---|---|---|---|---|

## Fig. 6

Fig. 7

survival_status

Survival Time [months]
training set (#88 patients)
pathology_M_stage="m0"

Number at risk
Group: <=threshold (0)

| 37 | 22 | 11 | 6 | 3 | 2 | 1 | 0 | 0 | 0 |

Group: >threshold (0)

| 51 | 15 | 8 | 2 | 2 | 2 | 1 | 1 | 1 | 0 |

survival_status

Survival Time [months]
testing set (#82 patients)
pathology_M_stage="m0"

Number at risk
Group: <=threshold (0)

| 44 | 22 | 15 | 10 | 3 | 2 | 2 | 2 | 2 | 0 |

Group: >threshold (0)

| 38 | 15 | 6 | 4 | 3 | 2 | 1 | 1 | 0 | 0 |

# Fig. 8

BCa Specific Death

Fig. 9

Fig. 10

Fig. 11

overall_survival

invasiveness="NMIBC"; TCGA training samples excluded

Number at risk
Group: <=threshold (0)
68        62        46        34        23        11        10        0
Group: >threshold (0)
35        29        16        10        4        3        2        0

overall_survival

invasiveness="NMIBC"; TCGA training samples excluded

Number at risk
Group: <=threshold (0.2)
76        70        50        35        23        11        9        0
Group: >threshold (0.2)
27        21        12        9        4        3        3        0

**Fig. 12**

Fig. 13

Fig. 14

Fig. 15

Fig. 16

**Fig. 17**

5-year cancer specific survial
exclude training samples

5-year overall survial
exclude training samples

**Fig. 18**

**Fig. 19**

Fig. 20

metastatic Urothelial Cancer
treated with atezolizumab

Number at risk
Group: I
118  98  77  64  52  45  39  27  23  7  0
Group: II
95  70  59  49  45  39  36  27  21  1  0
Group: III
69  53  43  32  27  25  20  15  10  4  0
Group: IV
66  55  44  38  32  28  24  19  12  4  0

metastatic Urothelial Cancer
treated with atezolizumab

Number at risk
Group: Basal/SCC-like
66  48  38  29  25  22  19  15  12  4  0
Group: Genomically unstable
70  56  49  44  41  40  37  27  20  4  0
Group: Infiltrated
92  78  66  55  46  37  30  21  13  2  0
Group: UroA
102  80  59  47  39  34  29  22  19  6  0
Group: UroB
18  14  11  8  5  4  4  3  2  0  0

# Fig. 21

Fig. 22

survival_status

**Fig. 23**

metastatic Urothelial Cancer
treated with atezolizumab

Number at risk
Group: Complete/Partial Response
31   31   31   31   31   31   29   27   24   4   0
Group: Stable/Progressive Disease
44   43   37   31   28   25   20   12   7   3   0

metastatic Urothelial Cancer
treated with atezolizumab

Number at risk
Group: Complete/Partial Response
21   21   21   21   20   19   18   9   8   2   0
Group: Stable/Progressive Disease
175   143   104   74   53   38   28   18   14   3   0

## Fig. 24

Fig. 25

Fig. 26

training set

testing set

**Fig. 27**

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 22 19 6480

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/104147 A1 (INST PAOLI CALMETTES [FR]) 14 June 2018 (2018-06-14)<br>* abstract; claim 1 *<br>* page 20; figure 2; table 7 * | 1-15 | INV.<br>C12Q1/6886 |
| A | YIN HUBIN ET AL: "Identification of a 13-mRNA signature for predicting disease progression and prognosis in patients with bladder cancer",<br>ONCOLOGY REPORTS,<br>12 December 2019 (2019-12-12),<br>XP093024248,<br>ISSN: 1021-335X, DOI: 10.3892/or.2019.7429<br>* the whole document * | 1-15 | |
| X | WO 2022/069201 A1 (KONINKLIJKE PHILIPS NV [NL]) 7 April 2022 (2022-04-07)<br>* abstract; claims 1, 3, 5, 11, 13-15; figures 4-5 *<br>* page 87, lines 22-26 *<br>* page 36, lines 27-30 *<br>* page 88, lines 3-5 *<br>* page 43, lines 12-19 *<br>* page 2, lines 28-33 * | 1-15 | |

**TECHNICAL FIELDS SEARCHED (IPC)**

C12Q

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 19 April 2023 | Aguilera, Miguel |

EPO FORM 1503 03.82 (P04E07)

Europäisches
Patentamt

European
Patent Office

Office européen
des brevets

**INCOMPLETE SEARCH
SHEET C**

```
Claim(s) searched incompletely:
        1-15

Reason for the limitation of the search:

The reasons for which a complete meaningful search is not possible are
specified in the annexed provisional opinion accompanying the partial
search results (EPO Form 1707).
```

Europäisches
Patentamt

European
Patent Office

Office européen
des brevets

**Application Number**

**EP 22 19 6480**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-15(partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

Application Number

**EP 22 19 6480**

The Search Division considers that the present European patent application does not comply with the requirements of unity of invention and relates to several inventions or groups of inventions, namely:

1. claims: 1-15(partially)

   Method of predicting an outcome of a bladder cancer subject, comprising determining or receiving the result of a determination of a gene expression profile comprising comprising ALL the 14 gene expression markers listed in claim 1 as "immune defence response" genes. Computer program product comprising instructions to carry out the above method. Use of a diagnostic kit comprising primers or probes suitable for carrying out said method.
   ---

2. claims: 1-15(partially)

   Method of predicting an outcome of a bladder cancer subject, comprising determining or receiving the result of a determination of a gene expression profile comprising comprising ALL the 17 gene expression markers listed in claim 1 as "T-cell receptor signalling" genes. Computer program product comprising instructions to carry out the above method. Use of a diagnostic kit comprising primers or probes suitable for carrying out said method.
   ---

3. claims: 1-15(partially)

   Method of predicting an outcome of a bladder cancer subject, comprising determining or receiving the result of a determination of a gene expression profile comprising comprising ALL the 8 gene expression markers listed in claim 1 as "PDE4D7 correlated" genes. Computer program product comprising instructions to carry out the above method. Use of a diagnostic kit comprising primers or probes suitable for carrying out said method.
   ---

## ANNEX TO THE EUROPEAN SEARCH REPORT
## ON EUROPEAN PATENT APPLICATION NO.

EP 22 19 6480

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2023

| Patent document cited in search report | | Publication date | Patent family member(s) | | Publication date |
|---|---|---|---|---|---|
| WO 2018104147 | A1 | 14-06-2018 | EP | 3333268 A1 | 13-06-2018 |
| | | | TW | 201827603 A | 01-08-2018 |
| | | | WO | 2018104147 A1 | 14-06-2018 |
| WO 2022069201 | A1 | 07-04-2022 | CN | 116249788 A | 09-06-2023 |
| | | | EP | 3978629 A1 | 06-04-2022 |
| | | | EP | 4222286 A1 | 09-08-2023 |
| | | | WO | 2022069201 A1 | 07-04-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MANTOVANI et al.** *Nature,* 2008, vol. 454 (7203), 436-44
- **GIRALDO et al.** *Br J Cancer.,* 2019, vol. 120 (1), 45-53
- **GIRALDO BR.** *J Cancer,* 2019, vol. 120 (1), 45-53
- **GASSER S. et al.** Sensing of dangerous DNA. *Mechanisms of Aging and Development,* 2017, vol. 165, 33-46
- **MOSENDEN R. ; TASKEN K.** Cyclic AMP-mediated immune regulation - Overview of mechanisms of action in T-cells. *Cell Signal,* 2011, vol. 23 (6), 1009-1016
- **TASKEN K. ; RUPPELT A.** Negative regulation of T-cell receptor activation by the cAMP-PKA-Csk signalling pathway in T-cell lipid rafts. *Front Biosci,* 2006, vol. 11, 2929-2939
- Immunity Leashed - Mechanisms of Regulation in the Human Immune System. **TORHEIM E.A.** Thesis for the degree of Philosophiae Doctor (PhD), The Biotechnology Centre of Ola. University of Oslo, 2009
- **TASKEN et al.** *Front in Bioscience,* 2006, vol. 11, 2929-2939
- **MOSENDEN et al.** *Cell Signalling,* 2011, vol. 23, 1009-1016
- **ABRAHAMSEN H. E.** TCR- and CD28-mediated recruitment of phosphodiesterase 4 to lipid rafts potentiates TCR signaling. *J Immunol,* 2004, vol. 173, 4847-4848
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384